# EUROPÄISCHE PATENTSCHRIFT

(11) **EP 1 410 803 B1**
(45) Veröffentlichungstag und Bekanntmachung des Hinweises auf die Patenterteilung: **29.07.2009**
(21) Anmeldenummer: 03023591.5
(22) Anmeldetag: 16.10.2003
(51) Int. Cl.: A61K 38/17, C07K 14/47, C07K 16/18, C12Q 1/68, C12N 15/11, A01K 67/027, C12N 5/10

(54) **MIA-2 Proteine**
MIA-2 proteins
Protéines MIA-2

(30) Priorität: 16.10.2002 DE 10248248; 07.02.2003 DE 10305082
(43) Veröffentlichungstag der Anmeldung: 21.04.2004
(73) Patentinhaber: Scil Proteins GmbH, 06120 Halle/Saale (DE)
(72) Erfinder: Bosserhoff, Anja, 93053 Regensburg (DE); Hellerbrand, Claus, 93053 Regensburg (DE); Büttner, Reinhard, 53343 Wachtberg-Pech (DE)
(74) Vertreter: Behnisch, Werner

(56) Entgegenhaltungen:
- EP-A- 0 909 954
- WO-A-01/70253
- WO-A-01/75068
- BOSSERHOFF ANJA K ET AL: "Specific expression and regulation of the new melanoma inhibitory activity-related gene MIA2 in hepatocytes." JOURNAL OF BIOLOGICAL CHEMISTRY, Bd. 278, Nr. 17, 25. April 2003 (2003-04-25), Seiten 15225-15231, XP002266135 ISSN: 0021-9258
- DATABASE EMBL [Online] 24. Februar 2003 (2003-02-24) BOSSERHOFF AK ET AL.: "Homo sapiens melanoma inhibitory activity protein 2 mRNA, complete cds." retrieved from EBI Database accession no. AF390175 XP002266137
- DATABASE EMBL [Online] 24. Februar 2003 (2003-02-24) BOSSERHOFF AK ET AL.: "Mus musculus melanoma inhibitory activity protein 2 mRNA, complete cds." retrieved from EBI Database accession no. AF390177 XP002266138
- DATABASE EMBL [Online] Sequence version 1 / Sequence Archive, 2. November 2001 (2001-11-02) BOSSERHOFF ET AL. : "Mus musculus melanoma inhibitory activity protein 2 mRNA, complete cds." retrieved from EBI Database accession no. AF390177 XP002266139
- DATABASE EMBL [Online] Sequence version 1 / Sequence archive, 2. November 2001 (2001-11-02) BOSSERHOFF AK ET AL. : "Homo sapiens melanoma inhibitory activity protein 2 mRNA, complete cds." retrieved from EBI Database accession no. AF390175.1 XP002266140
- BOSSERHOFF A -K ET AL: "Expression, function and clinical relevance of MIA (Melanoma Inhibitory Activity)" HISTOLOGY AND HISTOPATHOLOGY, Bd. 17, Nr. 1, Januar 2002 (2002-01), Seiten 289-300, XP008026088 ISSN: 0213-3911
- DATABASE EMBL [Online] 18. Oktober 1999 (1999-10-18) BIRREN B. ET AL.: "Homo sapiens clone RP11-15H22, WORKING DRAFT SEQUENCE, 23 unordered pieces." retrieved from EBI Database accession no. AC011779 XP002266141
- CEREDIG RHODRI ET AL: "Fetal liver organ cultures allow the proliferative expansion of pre-B receptor-expressing pre-B-II cells and the differentiation of immature and mature B cells in vitro" INTERNATIONAL IMMUNOLOGY, Bd. 10, Nr. 1, Januar 1998 (1998-01), Seiten 49-59, XP002266136 ISSN: 0953-8178

### SEQUENZPROTOKOLL

<110> Scil Proteins GmbH
<120> MIA-2 Protein
<130> P16412
<150> DE 10248248.9
   <151> 2002-10-16
<150> DE 10305082.5
   <151> 2003-02-07
<160> 36
<170> Patent In version 3.1
<210> 1
   <211> 1626
   <212> DNA
   <213> Homo sapiens
<400> 1
   atggcaaaat ttggcgttca cagaatcctt cttctggcta tttctctgac aaagtgtctg     60
   gagagtacaa aactgctggc agaccttaaa aaatgtggtg acttggaatg tgaagcttta    120
   ataaacagag tctcagccat gagagattat agaggacctg actgccgata cctgaacttc    180
   actaagggag aagagatatc tgtttatgtt aaacttgcag gagaaaggga agatttgtgg    240
   gcaggaaaag gaaaggagtt tggatatttt cccagagatg cagtccagat tgaagaggtg    300
   ttcatatctg aggaaattca gatgtcaacg aaagaatctg actttctttg tcttcttgga    360
   gtaagttaca catttgacaa tgaagatagt gaattaaacg gtgattatgg tgaaaatata    420
   tatccttatg aagaagataa agatgaaaaa tctagtatat atgaaagtga ttttcagata    480
   gaacctggat tttatgcaac ttatgaaagt actttgtttg aagaccaagt tccagcatta    540
   gaggctcctg aagatatcgg aagtaccagt gaatcaaaag actgggaaga agtagttgtt    600
   gaaagtatgg aacaggatcg tattccagaa gtgcatgtcc caccatcttc agctgtgtct    660
   ggagtcaaag aatggtttgg attgggagga gaacaagctg aagagaaggc ttttgaatca    720
   gttattgaac ctgtacaaga aagctcattt cggagtagaa aaatagcagt ggaagatgag    780
   aatgacctag aggaattaaa taatggtgag cctcaaacag aacatcagca agaatctgaa    840
   tcagaaattg attcagtgcc aaagacacag tctgaactag catctgagtc agagcacatt    900
   cccaaacctc aatccactgg ttggtttggt ggaggattta caagttattt aggttttgga    960
   gatgaggata cagggcttga attaatagct gaagaaagca atccaccact acaagatttt   1020
   cccaattcca tatcatctga taaagaagcc acagttccat gtacagaaat attaacagaa   1080
   aaaaaagaca caatcactaa tgatagcttg agtctcaagc caagttggtt tgattttggt   1140
   tttgctatac taggctttgc atatgccaag gaagataaaa ttatgttaga tgacaggaaa   1200
   aatgaagaag atggtggggc agatgaacat gaacatcctc taacaagtga attagaccct   1260
   gaaaaagaac aagaaataga aacgataaaa attatagaaa cagaagatca aatagacaag   1320
   aaaccagtct cagaaaaaac agacgaatct gatactatac catatttgaa aaagttcttg   1380
   tataattttg acaacccttg gaacttccag aacattccaa aggaaacaga attgccattt   1440
   cccaaacaga tactggatca aaataatgta attgaaaatg aagaaactgg agaattttcc   1500
   attgataatt atcccacaga taatacaaaa gttatgatat tcaaaagttc atacagtctg   1560
   tcaggttggt atgaaaatat ttacattaga atttatttta tttttaaaca taatttattt   1620
   ttataa                                                              1626
<210> 2
   <211> 3090
   <212> DNA
   <213> Homo sapiens
<400> 2
   tagcacgagt gtgggagcat ttctaatagg agcaaatctc tccagaccgg catcttaggc     60
   aaaaattttt cttgaccgaa atctccctca ggccaatgaa taccaaattt agattttgct    120
   gggcccttgc tgttcttaac ttagaacatg attaagtcaa ttacttctga atttatccat    180
   aaattcttta tattgtcttt aatatttaat acaaaaacaa agagagttaa actacattaa    240
   ttactaaatg ttcttgattt aatactggat ttggtaatat aaaatatgca aataaccctg    300
   agctaaattt tgacgctgaa gagctgatcc ttctttaaat gtccttaaca acacagcttg    360
   tagatttgca aaggtactca agaaactaac ctacatcttt tctaaaaaaa tagagacagg    420
   gtctcactct gtcacgcagg gtggcatgca gtggtgtgac aatagctcac tcagcctcaa    480
   actcctgggg tcaagcaatc ctcctgcctc agtctcccaa agtgttggga ttacagttgt    540
   gaggcacctg gcctaaccta cacctttttg tgtatgtatg catacaactg tgcagtgctc    600
   tgttgcaagc caaattatcc tgaaatgcat gctctatcag attcttttga aggatatcaa    660
   aatcttagct ctgagagagc gggcttccta ctgatcattg ttttttgaaa acaaaccaac    720
   aaacaaactt cctttactta ggatcttgaa agcaaaagtc cttccttcac tttagcttgc    780
   tcgggggctg ggtatttgtc ctatttgtgt caggcactag aggggagtgt gacatcatac    840
   aagtgctggg ttctgaccta agaatcagaa gaccaactgt gtgctcactg atatgcccaa    900
   atacagtgaa aaagtacagc aaaggagggc ttagacaaat attttcccaa gaatgaaata    960
   ggacaaccat ggtttctgaa aatcgactga gacctacagt tttacccaaa aatttccaat   1020
   gtgggatttt gatccttttt gagtgacggt aaccttcaaa tgactggatt ttcatagctt   1080
   tattttagta tttatttatt tattttttca ttattgttat ttttttgaga tggactctct   1140
   ctctgttgcc caggctggag tgcagtggcg caatctcagc tcactgtaac ctttgcctcc   1200
   tgggctcaag cgattttcct acctcagcct cccaagtagc tgggattaca agtgcctgcc   1260
   acaacacctg gctgattttt gtatttttag tagagatggg gttttaccat attggccagg   1320
   ctggtcttga actcctgacc tcaggtgatc ctcctgcctt ggctttccaa agtgctggga   1380
   ttacaggtat gagccaccgg gcccggctga tttatttttt tatttttatt ttttaatttt   1440
   atttatttat ttttttgaga cggggtctcg ctctgtcgcc caggctagag tgcagtggca   1500
   agatcttggc tcactgcaag ctccgcctcc caggttcagg ccattctcct gcctcagcct   1560
   tccgagtagc tgggaataca ggcgcccgac accactcctg gctaattttt tgtatttttt   1620
   agtagagacg gggtttcact gtgttagcca ggatggtctc gatctcctga cttcatgatc   1680
   cacccacctc ggccttccaa agtgctggga ttacagatgt gagccaccgc gcccagctga   1740
   tttattttaa aataatatac gggccaggtg tggtgactca tgcttgtaat cccagcactt   1800
   tgggaggctg aagcaggagg attacttgag tccaggagtt caagatcagc ctggccaaca   1860
   cagtgagacc tcatctctac taaaaataaa atattagcca gctgtgtccc gcatgcctgt   1920
   ggttcagcta ctcaggaagg ggaggtggaa ggattgctca agcctgggag gcccgcctgg   1980
   gctagagtaa gactctgtct caaaaacaaa acaaaactaa aaaagacata tgctttcttt   2040
   tttaaaaaag aaagccaaac aatataggaa actacaaaga agaaaataaa taccaacgga   2100
   aatcacactg tatacttcta ccatgtgggg gggcacttat tcagatatct ctctttcatt   2160
   ctctgtccac ttaaagaaat atgcatacag tttttaaagc atggagtaat actctacctg   2220
   ctgtttctgg agtagtattg ttgtagacag agtgacctct ttcaatggca gatgacttga   2280
   ttcttggtgg taaagcaagt ggttggttat aagtatgggg tctcattgtg cctaggttaa   2340
   catttctttt ccactcatat gtgccattga gtaagtgatt taacccatct ctctgtgcct   2400
   cagtttcctt atacacaaaa gtggggacga taatattacc tacctcagag agttggtgtg   2460
   aaggttatat cagatcatat atttaagtac ttacttaaga cagtacctgg aacatagtaa   2520
   gtacttacta catttcaact attgttctta actaaataag ttttaacttc atagtaacaa   2580
   taaggggttt ttaaatagaa aagtggaatc tttttaaaaa tacttttcac catatttcaa   2640
   agccattctt ttaacataaa ttattactct ttttgcacac agctatatac gttatccact   2700
   gaaattagta tttgcaggta actacccttt tgcggaggcc tacttaaaaa ctatcctgtt   2760
   gaacacaagg tacctttaag tgagacagtt ttacatgggc ttcctttata acacattagt   2820
   ccaactaaaa atccttgtta attattaaac ccttaggtat tacaggaata cctgacactg   2880
   cagattgaaa acagacagtg tttgtctctc aagttaaacc aacaagccga tagaaaaagg   2940
   tagttatcaa gagattttta aaacttcaac cctttttctc ttatagttag tgaagagagt   3000
   agaatatctc cagttttggc tgacatctct acaacctgaa caattggctt aaacttcact   3060
   tgggattccc ggttgcttgt tttagcatgg 3090
<210> 3
   <211> 19
   <212> DNA
   <213> Homo sapiens
<400> 3
   atggcaaaat ttggcgttc 19
<210> 4
   <211> 26
   <212> DNA
   <213> Homo sapiens
<400> 4
   taaaaataaa ttatgtttaa aaataa 26
<210> 5
   <211> 541
   <212> PRT
   <213> Homo sapiens
<400> 5
   Met Ala Lys Phe Gly Val His Arg Ile Leu Leu Leu Ala Ile Ser Leu
   1               5                   10                  15
   Thr Lys Cys Leu Glu Ser Thr Lys Leu Leu Ala Asp Leu Lys Lys Cys
               20                  25                  30
   Gly Asp Leu Glu Cys Glu Ala Leu Ile Asn Arg Val Ser Ala Met Arg
           35                  40                  45
   Asp Tyr Arg Gly Pro Asp Cys Arg Tyr Leu Asn Phe Thr Lys Gly Glu
       50                  55                  60
   Glu Ile Ser Val Tyr Val Lys Leu Ala Gly Glu Arg Glu Asp Leu Trp
   65                  70                  75                  80
   Ala Gly Lys Gly Lys Glu Phe Gly Tyr Phe Pro Arg Asp Ala Val Gln
                   85                  90                  95
   Ile Glu Glu Val Phe Ile Ser Glu Glu Ile Gln Met Ser Thr Lys Glu
               100                 105                 110
   Ser Asp Phe Leu Cys Leu Leu Gly Val Ser Tyr Thr Phe Asp Asn Glu
           115                 120                 125
   Asp Ser Glu Leu Asn Gly Asp Tyr Gly Glu Asn Ile Tyr Pro Tyr Glu
       130                 135                 140
   Glu Asp Lys Asp Glu Lys Ser Ser Ile Tyr Glu Ser Asp Phe Gln Ile
   145 150 155 160
   Glu Pro Gly Phe Tyr Ala Thr Tyr Glu Ser Thr Leu Phe Glu Asp Gln
   165 170 175
   Val Pro Ala Leu Glu Ala Pro Glu Asp Ile Gly Ser Thr Ser Glu Ser
   180 185 190
   Lys Asp Trp Glu Glu Val Val Val Glu Ser Met Glu Gln Asp Arg Ile
   195 200 205
   Pro Glu Val His Val Pro Pro Ser Ser Ala Val Ser Gly Val Lys Glu
   210 215 220
   Trp Phe Gly Leu Gly Gly Glu Gln Ala Glu Glu Lys Ala Phe Glu Ser
   225 230 235 240
   Val Ile Glu Pro Val Gln Glu Ser Ser Phe Arg Ser Arg Lys Ile Ala
   245 250 255
   Val Glu Asp Glu Asn Asp Leu Glu Glu Leu Asn Asn Gly Glu Pro Gln
   260 265 270
   Thr Glu His Gln Gln Glu Ser Glu Ser Glu Ile Asp Ser Val Pro Lys
   275 280 285
   Thr Gln Ser Glu Leu Ala Ser Glu Ser Glu His Ile Pro Lys Pro Gln
   290 295 300
   Ser Thr Gly Trp Phe Gly Gly Gly Phe Thr Ser Tyr Leu Gly Phe Gly
   305 310 315 320
   Asp Glu Asp Thr Gly Leu Glu Leu Ile Ala Glu Glu Ser Asn Pro Pro
   325 330 335
   Leu Gln Asp Phe Pro Asn Ser Ile Ser Ser Asp Lys Glu Ala Thr Val
   340 345 350
   Pro Cys Thr Glu Ile Leu Thr Glu Lys Lys Asp Thr Ile Thr Asn Asp
   355 360 365
   Ser Leu Ser Leu Lys Pro Ser Trp Phe Asp Phe Gly Phe Ala Ile Leu
   370 375 380
   Gly Phe Ala Tyr Ala Lys Glu Asp Lys Ile Met Leu Asp Asp Arg Lys
   385 390 395 400
   Asn Glu Glu Asp Gly Gly Ala Asp Glu His Glu His Pro Leu Thr Ser
   405 410 415
   Glu Leu Asp Pro Glu Lys Glu Gln Glu Ile Glu Thr Ile Lys Ile Ile
   420 425 430
   Glu Thr Glu Asp Gln Ile Asp Lys Lys Pro Val Ser Glu Lys Thr Asp
   435 440 445
   Glu Ser Asp Thr Ile Pro Tyr Leu Lys Lys Phe Leu Tyr Asn Phe Asp
   450 455 460
   Asn Pro Trp Asn Phe Gln Asn Ile Pro Lys Glu Thr Glu Leu Pro Phe
   465 470 475 480
   Pro Lys Gln Ile Leu Asp Gln Asn Asn Val Ile Glu Asn Glu Glu Thr
   485 490 495
   Gly Glu Phe Ser Ile Asp Asn Tyr Pro Thr Asp Asn Thr Lys Val Met
   500 505 510
   Ile Phe Lys Ser Ser Tyr Ser Leu Ser Gly Trp Tyr Glu Asn Ile Tyr
   515 520 525
   Ile Arg Ile Tyr Phe Ile Phe Lys His Asn Leu Phe Leu
   530 535 540
<210> 6
   <211> 1554
   <212> DNA
   <213> Mus
<400> 6
   atggcggaag tcagtgttca aagaatcctt cttttggttg tttctctggc caagtgtctg      60
   gagggtacaa agttgctggc acaccttaag aagtgtggtg acttggaatg tgaaactttg     120
   atcagccgag tcttagccct gagagattac acaggacccg actgtcggta cctgaacttc     180
   actacgggag aagagatatc tgtttatgtt aaacttggag gagacagaga agatttgtgg     240
   gcaggaagca aaggaaaaga ctttggattt tttcccagag atgcagtcga gattgaagag     300
   gtgttcatat ctgaagaagt cgaaatgcca actaaatctg actttctttg tcttcttgga     360
   gaaggctaca tatttggaag tgaacagagt gaattaaaca gtgaagatga tgaagaacat     420
   atgtacccat atgaaaaaga tgaagaccaa aactataata tatatgaggg tgattttcag     480
   ccagaacctg acttatatgc agctgctgaa gggactttgt tggaggacca aattccagca     540
   tccgaagctc ctgatgattt ccgattctcc agtgagtgga aggcctggga aggggctgga     600
   agccagggtg gaggggagca ggattacact gcagactctg accaagactt gccatccctc     660
   agtaagccag aaaggcaagg atggtttggc ctggggacag aagaagctga agagaaggtt     720
   ttcgaatcag atactgaacc tacacaagaa ttagcactag aagaggagag tgacctggag     780
   aaattacaca gtggcgaacc ccaagtggaa cttgagcaag agccaaaatc agagacatta     840
   gaattcagtt cagtgccgga cgaagagtat gagctagaat ctgagacgga gagtatcctc     900
   aaaccccaag cttctggctg gtttggtgag ggccttacaa gttatttagg ttttggaaat     960
   gaggaggcag gacttgagtt attgtccaaa gaaagcaatc caccattaca agatattccc    1020
   agctctgttc caccagatga agaagtcccg gctccatgca gagaaatctc aacagacaag    1080
   gaagatgcag tcattaatga tagctcggtt ctcagtccaa gctggtttta ctatggattt    1140
   ggtatgctag gctttacaaa tgccgacgaa gacaacattg tttcagacaa aggagaaaat    1200
   gaagatggtg aggtagataa cctcaaacat cctataggaa gtgactttga ccctgaaaag    1260
   gaacaagaaa ggaaaatagt aactgtggaa accgaagacc aggcaggtac agaaagcgtc    1320
   ttggagaaga cagacgagtc tggttccatg cagtatctga agaagttctt tgataatcct    1380
   tggggcttcc agagtctccc agaggacaca gaattaccat tttccaaaaa gatgctggat    1440
   caagatgata tagtagaaaa tgacaaaatt gaagaacttt ccactgaaaa ttctcccaca    1500
   ggtagcatga aagaccccgt gatgctggcg agcagatacg ttctgtcagg ttag          1554
<210> 7
   <211> 18
   <212> DANN
   <213> Mus
<400> 7
   cctgcccaca aatcttct 18
<210> 8
   <211> 850
   <212> DNA
   <213> Homo sapiens
<400> 8
   ttgtagacag agtgacctct ttcaatggca gatgacttga ttcttggtgg taaagcaagt 60
   ggttggttat aagtatgggg tctcattgtg cctaggttaa catttctttt ccactcatat 120
   gtgccattga gtaagtgatt taacccatct ctctgtgcct cagtttcctt atacacaaaa 180
   gtggggacga taatattacc tacctcagag agttggtgtg aaggttatat cagatcatat 240
   atttaagtac ttacttaaga cagtacctgg aacatagtaa gtacttacta catttcaact 300
   attgttctta actaaataag ttttaacttc atagtaacaa taaggggttt ttaaatagaa 360
   aagtggaatc tttttaaaaa tacttttcac catatttcaa agccattctt ttaacataaa 420
   ttattactct ttttgcacac agctatatac gttatccact gaaattagta tttgcaggta 480
   actacccttt tgcggaggcc tacttaaaaa ctatcctgtt gaacacaagg tacctttaag 540
   tgagacagtt ttacatgggc ttcctttata acacattagt ccaactaaaa atccttgtta 600
   attattaaac ccttaggtat tacaggaata cctgacactg cagattgaaa acagacagtg 660
   tttgtctctc aagttaaacc aacaagccga tagaaaaagg tagttatcaa gagattttta 720
   aaacttcaac cctttttctc ttatagttag tgaagagagt agaatatctc cagttttggc 780
   tgacatctct acaacctgaa caattggctt aaacttcact tgggattccc ggttgcttgt 840
   tttagcatgg 850
<210> 9
   <211> 20
   <212> DNA
   <213> Homo sapiens
<400> 9
   aattcactat cttcattgtc 20
<210> 10
   <211> 30
   <212> DNA
   <213> Homo sapiens
<400> 10
   gacagatctg ggtctcattg tgcctaggtt 30
<210> 11
   <211> 29
   <212> DNA
   <213> Homo sapiens
<400> 11
   gacagatcta cccatctctc tgtgcctca 29
<210> 12
   <211> 29
   <212> DNA
   <213> Homo sapiens
<400> 12
   gacagatctt cagagagttg gtgtgaagg 29
<210> 13
   <211> 29
   <212> DNA
   <213> Homo sapiens
<400> 13
   gacagatctc agggtctcac tctgtcacg 29
<210> 14
   <211> 29
   <212> DNA
   <213> Homo sapiens
<400> 14
   gacagatctg gaatacctga cactgcaga 29
<210> 15
   <211> 27
   <212> DNA
   <213> Homo sapiens
<400> 15
   gacaagctta caagcaaccg ggaatcc 27
<210> 16
   <211> 33
   <212> DNA
   <213> Homo sapiens
<400> 16
   gatccatatg ctggagagta caaaactgct ggc 33
<210> 17
   <211> 32
   <212> DNA
   <213> Homo sapiens
<400> 17
   gcatcggatc cttacaagac aaagaaagtc ag 32
<210> 18
   <211> 28
   <212> DNA
   <213> Homo sapiens
<400> 18
   gatgaattca tggcaaaatt tggcgttc 28
<210> 19
   <211> 29
   <212> DNA
   <213> Homo sapiens
<400> 19
   gatgaattct caaagacaaa gaaagtcag 29
<210> 20
   <211> 357
   <212> DNA
   <213> Homo sapiens
<400> 20
   atggcaaaat ttggcgttca cagaatcctt cttctggcta tttctctgac aaagtgtctg 60
   gagagtacaa aactgctggc agaccttaaa aaatgtggtg acttggaatg tgaagcttta 120
   ataaacagag tctcagccat gagagattat agaggacctg actgccgata cctgaacttc 180
   actaagggag aagagatatc tgtttatgtt aaacttgcag gagaaaggga agatttgtgg 240
   gcaggaaaag gaaaggagtt tggatatttt cccagagatg cagtccagat tgaagaggtg 300
   ttcatatctg aggaaattca gatgtcaacg aaagaatctg actttctttg tctttga 357
<210> 21
   <211> 20
   <212> DNA
   <213> Homo sapiens
<400> 21
   atggcaaaat ttggcgttca 20
<210> 22
   <211> 20
   <212> DNA
   <213> Homo sapiens
<400> 22
   ttataaaaat aaattatgtt 20
<210> 23
   <211> 360
   <212> DNA
   <213> Mus
<400> 23
   atggcaaaat ttggcgttca cagaatcctt cttttggttg tttctctggc caagtgtctg      60
   gagggtacaa agttgctggc acaccttaag aagtgtggtg acttggaatg tgaaactttg     120
   atcagccgag tcttagccct gagagattac acaggacccg actgtcggta cctgaacttc     180
   actacgggag aagagatatc tgtttatgtt aaacttggag gagacagaga agatttgtgg     240
   gcaggaagca aaggaaaaga ctttggatat tttcccagag atgcagtcca gattgaagag     300
   gtgttcatat ctgaggaaat tcagatgtca acgaaagaat ctgactttct ttgtctttaa     360
<210> 24
   <211> 25
   <212> DNA
   <213> Homo sapiens
<400> 24
   tggaatcctg tggcatccat gaaac 25
<210> 25
   <211> 25
   <212> DNA
   <213> Homo sapiens
<400> 25
   taaaacgcag ctcagtaaca gtccg 25
<210> 26
   <211> 18
   <212> DNA
   <213> Homo sapiens
<400> 26
   cctgcccaca aatcttcc 18
<210> 27
   <211> 1628
   <212> DNA
   <213> Mus musculus
<400> 27
   atggcggaag tcagtgttca aagaatcctt cttttggttg tttctctggc caagtgtctg 60
   gagggtacaa agttgctggc acaccttaag aagtgtggtg acttggaatg tgaaactttg 120
   atcagccgag tcttagccct gagagattac acaggacccg actgtcggta cctgaacttc 180
   actacgggag aagagatatc tgtttatgtt aaacttggag gagacagaga agatttgtgg 240
   gcaggaagca aaggaaaaga ctttggattt tttcccagag atgcagtcga gattgaagag 300
   gtgttcatat ctgaagaagt cgaaatgcca actaaatctg actttctttg tcttcttgga 360
   gaaggctaca tatttggaag tgaacagagt gaattaaaca gtgaagatga tgaagaacat 420
   atgtacccat atgaaaaaga tgaagaccaa aactataata tatatgaggg tgattttcag 480
   ccagaacctg acttatatgc agctgctgaa gggactttgt tggaggacca aattccagca 540
   tccgaagctc ctgatgattt ccgattctcc agtgagtgga aggcctggga aggggctgga 600
   agccagggtg gaggggagca ggattacact gcagactctg accaagactt gccatccctc 660
   agtaagccag aaaggcaagg atggtttggc ctggggacag aagaagctga agagaaggtt 720
   ttcgaatcag atactgaacc tacacaagaa ttagcactag aagaggagag tgacctggag 780
   aaattacaca gtggcgaacc ccaagtggaa cttgagcaag agccaaaatc agagacatta 840
   gaattcagtt cagtgccgga cgaagagtat gagctagaat ctgagacgga gagtatcctc 900
   aaaccccaag cttctggctg gtttggtgag ggccttacaa gttatttagg ttttggaaat 960
   gaggaggcag gacttgagtt attgtccaaa gaaagcaatc caccattaca agatattccc 1020
   agctctgttc caccagatga agaagtcccg gctccatgca gagaaatctc aacagacaag 1080
   gaagatgcag tcattaatga tagctcggtt ctcagtccaa gctggtttta ctatggattt 1140
   ggtatgctag gctttacaaa tgccgacgaa gacaacattg tttcagacaa aggagaaaat 1200
   gaagatggtg aggtagataa cctcaaacat cctataggaa gtgactttga ccctgaaaag 1260
   gaacaagaaa ggaaaatagt aactgtggaa accgaagacc aggcaggtac agaaagcgtc 1320
   ttggagaaga cagacgagtc tggttccatg cagtatctga agaagttctt tgataatcct 1380
   tggggcttcc agagtctccc agaggacaca gaattaccat tttccaaaaa gatgctggat 1440
   caagatgata tagtagaaaa tgacaaaatt gaagaacttt ccactgaaaa ttctcccaca 1500
   ggtagcatga aagaccccgt gatgctggcg agcagatacg ttctgtcagg ttagtatgaa 1560
   agacttatta atgcacttgt taagatcatt tatttttcta agtccaaatc agatgaagat 1620
   ttcgtgct 1628
<210> 28
   <211> 517
   <212> PRT
   <213> Mus musculus
<400> 28
   Met Ala Glu Val Ser Val Gln Arg Ile Leu Leu Leu Val Val Ser Leu
   1               5                   10                  15
   Ala Lys Cys Leu Glu Gly Thr Lys Leu Leu Ala His Leu Lys Lys Cys
               20                  25                  30
   Gly Asp Leu Glu Cys Glu Thr Leu Ile Ser Arg Val Leu Ala Leu Arg
           35                  40                  45
   Asp Tyr Thr Gly Pro Asp Cys Arg Tyr Leu Asn Phe Thr Thr Gly Glu
       50                  55                  60
   Glu Ile Ser Val Tyr Val Lys Leu Gly Gly Asp Arg Glu Asp Leu Trp
   65                  70                  75                  80
   Ala Gly Ser Lys Gly Lys Asp Phe Gly Phe Phe Pro Arg Asp Ala Val
                   85                  90                  95
   Glu Ile Glu Glu Val Phe Ile Ser Glu Glu Val Glu Met Pro Thr Lys
               100                 105                 110
   Ser Asp Phe Leu Cys Leu Leu Gly Glu Gly Tyr Ile Phe Gly Ser Glu
           115                 120                 125
   Gln Ser Glu Leu Asn Ser Glu Asp Asp Glu Glu His Met Tyr Pro Tyr
       130                 135                 140
   Glu Lys Asp Glu Asp Gln Asn Tyr Asn Ile Tyr Glu Gly Asp Phe Gln
   145                 150                 155                 160
   Pro Glu Pro Asp Leu Tyr Ala Ala Ala Glu Gly Thr Leu Leu Glu Asp
                   165                 170                 175
   Gln Ile Pro Ala Ser Glu Ala Pro Asp Asp Phe Arg Phe Ser Ser Glu
   180 185 190
   Trp Lys Ala Trp Glu Gly Ala Gly Ser Gln Gly Gly Gly Glu Gln Asp
   195 200 205
   Tyr Thr Ala Asp Ser Asp Gln Asp Leu Pro Ser Leu Ser Lys Pro Glu
   210 215 220
   Arg Gln Gly Trp Phe Gly Leu Gly Thr Glu Glu Ala Glu Glu Lys Val
   225 230 235 240
   Phe Glu Ser Asp Thr Glu Pro Thr Gln Glu Leu Ala Leu Glu Glu Glu
   245 250 255
   Ser Asp Leu Glu Lys Leu His Ser Gly Glu Pro Gln Val Glu Leu Glu
   260 265 270
   Gln Glu Pro Lys Ser Glu Thr Leu Glu Phe Ser Ser Val Pro Asp Glu
   275 280 285
   Glu Tyr Glu Leu Glu Ser Glu Thr Glu Ser Ile Leu Lys Pro Gln Ala
   290 295 300
   Ser Gly Trp Phe Gly Glu Gly Leu Thr Ser Tyr Leu Gly Phe Gly Asn
   305 310 315 320
   Glu Glu Ala Gly Leu Glu Leu Leu Ser Lys Glu Ser Asn Pro Pro Leu
   325 330 335
   Gln Asp Ile Pro Ser Ser Val Pro Pro Asp Glu Glu Val Pro Ala Pro
   340 345 350
   Cys Arg Glu Ile Ser Thr Asp Lys Glu Asp Ala Val Ile Asn Asp Ser
   355 360 365
   Ser Val Leu Ser Pro Ser Trp Phe Tyr Tyr Gly Phe Gly Met Leu Gly
   370 375 380
   Phe Thr Asn Ala Asp Glu Asp Asn Ile Val Ser Asp Lys Gly Glu Asn
   385 390 395 400
   Glu Asp Gly Glu Val Asp Asn Leu Lys His Pro Ile Gly Ser Asp Phe
   405 410 415
   Asp Pro Glu Lys Glu Gln Glu Arg Lys Ile Val Thr Val Glu Thr Glu
   420 425 430
   Asp Gln Ala Gly Thr Glu Ser Val Leu Glu Lys Thr Asp Glu Ser Gly
   435 440 445
   Ser Met Gln Tyr Leu Lys Lys Phe Phe Asp Asn Pro Trp Gly Phe Gln
   450 455 460
   Ser Leu Pro Glu Asp Thr Glu Leu Pro Phe Ser Lys Lys Met Leu Asp
   465 470 475 480
   Gln Asp Asp Ile Val Glu Asn Asp Lys Ile Glu Glu Leu Ser Thr Glu
   485 490 495
   Asn Ser Pro Thr Gly Ser Met Lys Asp Pro Val Met Leu Ala Ser Arg
   500 505 510
   Tyr Val Leu Ser Gly
   515
<210> 29
   <211> 21
   <212> DNA
   <213> Homo sapiens
<400> 29
   gggagagacc ctttgaagtc a 21
<210> 30
   <211> 19
   <212> DNA
   <213> Homo sapiens
<400> 30
   aagaagatgg cggtggcat 19
<210> 31
   <211> 20
   <212> DNA
   <213> Homo sapiens
<400> 31
   cagtggagaa ggaacaagcg 20
<210> 32
   <211> 21
   <212> DNA
   <213> Homo sapiens
<400> 32
   ttggtggcag tttcagggat a 21
<210> 33
   <211> 21
   <212> DNA
   <213> Homo sapiens
<400> 33
   acaccaccta cctgaatgtc c 21
<210> 34
   <211> 20
   <212> DNA
   <213> Homo sapiens
<400> 34
   actctcccag ttgctccttg 20
<210> 35
   <211> 21
   <212> DNA
   <213> Homo sapiens
<400> 35
   gcccataata ccaccctgac a 21
<210> 36
   <211> 20
   <212> DNA
   <213> Homo sapiens
<400> 36
   tacagcctct tggcatcctc 20

## Beschreibung

Die vorliegende Erfindung betrifft das humane und murine MIA-2 Protein sowie die diese Proteine kodierenden Nukleinsäuren. Die Erfindung betrifft weiterhin Antikörper und Aptamere, die an diese Proteine binden sowie pharmazeutische und diagnostische Zusammensetzungen und deren Verwendung in der Therapie und Diagnose von Leberzirrhose, Leberfibrose oder von Lebertumoren/-Metastasen. Die vorliegende Erfindung betrifft weiterhin ein Verfahren zur Herstellung einer Organkultur sowie deren Verwendung zur Blutreinigung.

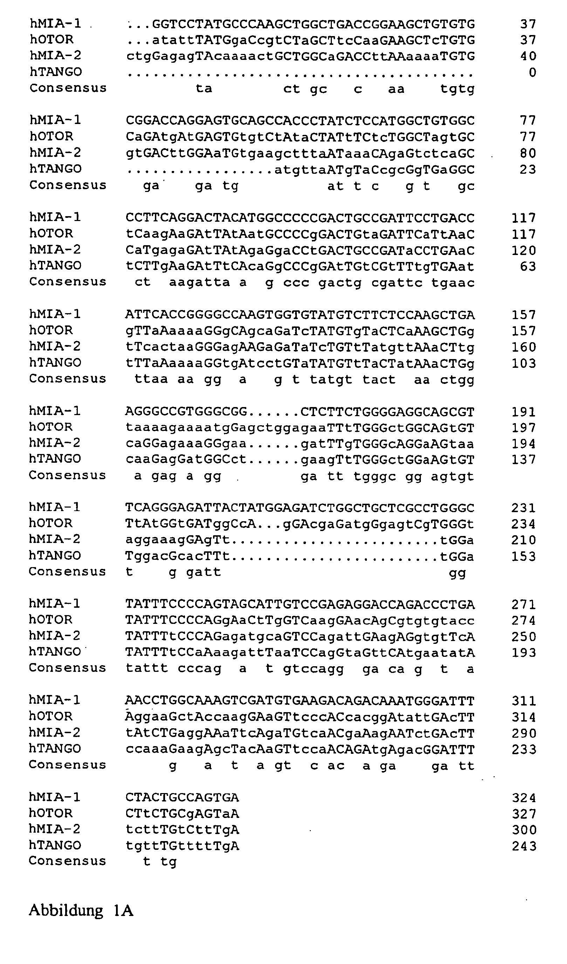

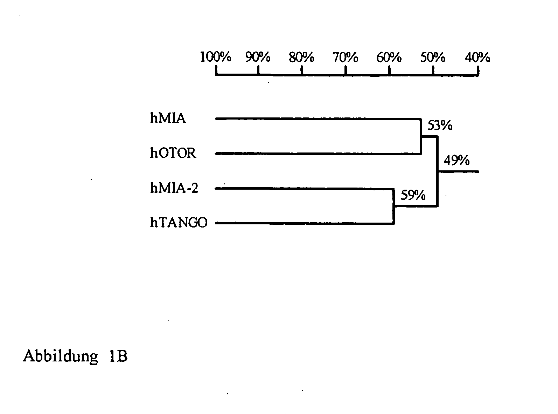

Das in Chondrozyten exprimierte Protein MIA ("melanoma inhibitory activity", auch CD-RAP "cartilage-derived retinoic acid-sensitive protein" genannt) wurde aufgrund seiner antiproliferativen Eigenschaften *in vitro* zunächst im Zellkulturüberstand von Melanomzellen nachgewiesen und isoliert. Nachdem das Protein gereinigt und ansequenziert worden war, konnte durch die Verwendung degenerierter Primer über RT-PCR ein Teil der humanen MIA cDNA kloniert werden. Dieses Fragment mit der Länge von 250 Nukleinsäureresten wurde schließlich in einem Phagenscreen als Sonde eingesetzt, um einen vollständig kodierenden cDNA Klon von MIA zu isolieren (Blesch et al., 1995). Eine Datenbanksuche mit dieser Sequenz zeigte damals keine bekannten homologen Gensequenzen. Heute sind die humane, murine, bovine, Ratten und die Zebrafisch Sequenz von MIA bekannt. Die Homologie innerhalb der Proteine ist sehr hoch, so daß es sich bei MIA um ein in der Evolution stark konserviertes Protein handelt (Abb.1).

Aus der ermittelten cDNA Sequenz konnte abgeleitet werden, daß MIA als ein 131 Aminosäuren Vorläuferprotein translatiert wird. Die Signalsequenz, eine hydrophobe Sequenz von 24 Aminosäuren, ist für den Transport des Proteins in das endoplasmatische Retikulum verantwortlich und wird dort abgespalten. Anschließend wird MIA von der Zelle in den Extrazellularraum sezerniert. Das reife Protein besteht aus 107 Aminosäuren und hat ein Molekulargewicht von ca. 11 kDa. Weitere Analysen der Proteinsequenz zeigten, daß MIA neben der Signalsequenz vier weitere stark hydrophobe Bereiche besitzt und, stabilisiert durch zwei intramolekulare Disulfidbrücken, eine globuläre Struktur ausbildet. Stellen, an denen MIA glykosyliert werden könnte (Asn-Gly-Ser/Thr; Ser-Gly), sind im Protein nicht nachweisbar, so daß eine N- oder O-Glykosylierung unwahrscheinlich ist.

Zur Klärung der Bedeutung von MIA in der Chondrozytenentwicklung und zur funktionellen Rolle wurden Labor MIA-defiziente Mäuse ("knock-outs") generiert (Moser et al., 2002 Mol Cell Biol. 2002 Mar; 22(5):1438-45).

MIA -/- Mäuse zeigen Störungen in der Organisation der Knorpelmatrix, deren Auswirkung auf die Integrität und Stabilität des Knorpels analysiert wird.

Vor kurzer Zeit wurde das MIA-homologe Protein OTOR (MIAL, FPD) (Cohen-Salmon et al., 2000; Rendtorff et al., 2001; Robertson et al., 2000) charakterisiert. OTOR zeigt eine sehr spezifische Expression in der Cochlea und im Auge. In durch die Erfinder durchgeführten Analysen konnte gezeigt werden, daß es in der MIA-defizienten Maus nicht zu einer vermehrten Expression von OTOR kommt (Moser et al., 2002 Mol Cell Biol. 2002 Mar; 22(5):1438-45).

Gegenstand der EP 0 909 954 ist ein Verfahren zur Diagnose von Knorpelerkrankungen durch den Nachweis von MIA, ein dafür geeignetes Reagenz, sowie die Verwendung von Antikörpern gegen MIA zum Nachweis von Knorpelerkrankungen. MIA-2 Sequenzen oder deren Verwendung sind hierin nicht erwähnt.

Ein Teil der humanen und murinen MIA-2-Sequenzen wurde bereits veröffentlicht (Bosserhoff, A.K. und Buettner, R; NCBI-Datenbank, November 2001 Bossehoff et al.: "Expression and Histopathology, Bd. 17, Nr. 1, January 2002, Seiten 289-300). Die Veröffentlichung umfasst jedoch lediglich die bp 1-354 der humanen MIA-2-Sequenz bzw. die bp 1- 357 der murinen MIA-2-Sequenz. Die bereits veröffentlichten Sequenzen enden jeweils mit dem Stoppcodon TAA.

Demgegenüber liegt der vorliegenden Erfindung die Aufgabe zu Grunde, eine MIA-2 Sequenz zur Verfügung zu stellen, die in vorteilhafter Weise zur Diagnose und Therapie verschiedener Leberschädigungen verwendet werden kann.

Diese Aufgabe wird durch den Gegenstand der unabhängigen Ansprüche gelöst. Vorteilhafte Ausführungsformen sind in den abhängigen Ansprüchen angegeben.

Überraschenderweise hat sich jetzt herausgestellt, dass die kodierende MIA-2 Sequenz und das daraus exprimierte Protein bei weitem länger als die bereits veröffentlichte Sequenz ist und es sich bei dem veröffentlichten Stoppkodon um einen Artefakt handelt. Unerwarteterweise gelang es den Erfindern, mit der vorliegenden Erfindung den bei weitem größten Teil des MIA-2 Proteins zu isolieren und zu sequenzieren.

Erste Versuche der Erfinder zeigten eine selektive Expression von MIA-2 in der murinen Leber. Diese leber-spezifische Expression konnte auch beim Menschen bestätigt werden. Nach detaillierterer Analyse konnte gezeigt werden, dass das Gen vornehmlich von Hepatozyten exprimiert wird.

Der MIA-2 Proteingehalt in der Leber dient sowohl als Maß für hepatische Gewebsschädigung, als auch als Maß für die Syntheseleistung. Versuche mit a) Zellüberständen von mit MIA-2 Expressionsplasmiden transfizierten Zellen und b) rekombinatem MIA-2 Protein zeigen auf hepatische Sternzellen eine antiproliferative und antifibrotische Wirkung (die Aktivierung hepatischer Sternzellen (HSZ) ist das Schlüsselereignis der hepatischen Fibrose und Zirrhose). Analoge Vorversuche mit Fibroblasten zeigen auch hier einen antiproliferativen und antifibrotischen Effekt. Dies weist auf einen generellen Wirkmechanismus und somit auf eine potentielle Anwendung auch in nicht-hepatischem Gewebe hin. Durch Stimulation mit unterschiedlichen Zytokinen oder unspezifische physikalische Reizung konnte in *in vitro* Modellen eine gesteigerte Expression von MIA-2 auf RNA Ebene in einer Hepatom-Zellinie nachgewiesen werden. Diese Versuche zeigen, daß durch ein verändertes Zytokin-Milieu oder verdrängendes Wachstum eventuell auch Lebertumoren/-Metastasen zu einer gesteigerten MIA-2 Expression führen können.

Es hat sich weiterhin überraschenderweise herausgestellt, dass MIA2 als diagnostischer Marker für die Akut-Phase von verschiedenen Erkrankungen (Infektionskrankheiten, entzündlichen Erkrankungen wie Morbus Crohn, Gastroenteritis, Gastritis, Spondylitis, Myokarditis, Gelenkrheumatismus und rheumatischen Erkrankungen) geeignet ist. Unter Infektionskrankheiten sind Krankheiten zu verstehen, die durch die Übertragung, Haftung, Eindringen und Vermehrung von Mikroorganismen in einen Makroorganismus entstehen. Im Rahmen der vorliegenden Erfindung besonders relevante Infektionskrankheiten sind Hepatitis, Tuberkulose, Malaria, Lungenentzündung, Borreliose, Influenza, Campylobacter-Infektionen, Gastroenteritis, Pocken, Anthrax und Typhus.

Während einer Infektion kommt es zu einer rapiden Erhöhung von bestimmten Proteinen im Serum, sogenannten Akut-Phase-Proteinen (APP). Die Konzentration dieser APP kann auf das 10-100fache der Norm ansteigen und bleibt während der Phase der Infektion erhöht. Eines der bekanntesten und in der Klinik weitverbreitetsten APP ist das C-reaktive Protein (CRP). Aus der Höhe und dem Verlauf der APP im Serum von Patienten können u.a. Rückschlüsse auf die Schwere und den Verlauf von (Infektions-)krankheiten gezogen werden. Auch bei einigen anderen Erkrankungen, wie chronisch entzündlichen Darmerkrankungen oder rheumatischen Erkrankungen finden sich APP bisweilen erhöht und konelieren mit der Schwere und dem Verlauf der Erkrankung.

Aus WO 01/75068 ist die Nukleotid- und Aminosäuresequenz für am728_60 bekannt. Das Dokument offenbart weiter, dass diese Aminosäuresequenz eine Verwandtschaft mit MIA aufweist. Jedoch werden keine Funktionen von am728_60 angegeben. Das Protein wurde aus Nierenzellen isoliert, eine leberspezifische Expression wird jedoch nicht beschrieben. Daneben werden Antikörper beschrieben, die gegen diese Sequenz gerichtet sind.

Die Erfinder haben jetzt herausgefunden, dass auch MIA2 ein APP ist und im Serum von Patienten als solches nachgewiesen werden kann.

Gemäß einem ersten Grundgedanken betrifft so die vorliegende Erfindung ein humanes MIA-2 Protein das durch eine leberspezifische Expression und eine antiproliferative und antifibrotische Wirkung gekennzeichnet ist und, das von der Nukleinsäure von SEQ ID NO. 1 oder Varianten hiervon kodiert wird, wobei die Varianten eine oder mehrere Substitutionen, Insertionen und/oder Deletionen im Vergleich zur Nukleinsäure von SEQ ID NO. 1 und eine antiproliferative und antifibrotische Wirkung aufweisen, vorausgesetzt dass:
a) diese Varianten unter stringenten Bedingungen an eine Nukleinsäure gemäß SEQ ID NO. 1 hybridisieren und weiterhin vorausgesetzt, dass diese Varianten ein Protein mit einer MIA-2-Aktivität kodieren, oder
b) diese Varianten Nukleinsäureveränderungen aufweisen, die auf die Degenerierung des genetischen Codes zurückzuführen sind, die für dieselbe oder eine funktionell äquivalente Aminosäure wie die Nukleinsäure von SEQ ID NO. 1 kodieren, und
c) das MIA-2-Protein jeweils nicht ausschließlich durch die Basen 1-354 von SEQ ID NO. 1 oder Bruchstücken hiervon kodiert wird.

Gemäß einem weiteren Grundgedanken stellt die vorliegende Erfindung ein murines MIA-2 Protein bereit das durch eine leberspezifische Expression und eine antiproliferative und antifibrotische Wirkung gekennzeichnet ist und, das von der Nukleinsäure von SEQ ID NO. 27 oder Varianten hiervon kodiert wird, wobei die Varianten eine oder mehrere Substitutionen, Insertionen und/oder Deletionen im Vergleich zur Nukleinsäure von SEQ ID NO. 27 und eine antiproliferative und antifibrotische Wirkung aufweisen, vorausgesetzt dass:
a) diese Varianten unter stringenten Bedingungen an eine Nukleinsäure gemäß SEQ ID NO. 27 hybridisieren und weiterhin vorausgesetzt, dass diese Varianten ein Protein mit einer MIA-2-Aktivität kodieren, oder
b) diese Varianten Nukleinsäureveränderungen aufweisen, die auf die Degenerierung des genetischen Codes zurückzuführen sind, die für dieselbe oder eine funktionell äquivalente Aminosäure wie die Nukleinsäure von SEQ ID NO. 27 kodieren, und
c) das MIA-2-Protein jeweils nicht ausschließlich durch die Basen 1-357 von SEQ ID NO. 27 oder Bruchstücken hiervon kodiert wird.

Die Erfindung betrifft weiterhin eine humane, isolierte Nukleinsäure, die die Nukleinsäure von SEQ ID NO. 1 oder Varianten hiervon umfasst, wobei die Varianten eine oder mehrere Substitutionen, Insertionen und/oder Deletionen im Vergleich zur Nukleinsäure von SEQ ID NO. 1 aufweisen und für ein Protein kochener das durch eine leberspezifische Expression und eine antiproliferative und antifibrotische Wirkung gekennzeichnet ist und vorausgesetzt dass:
a) diese Varianten unter stringenten Bedingungen an eine Nukleinsäure gemäß SEQ ID NO. 1 hybridieseren und weiterhin vorausgesetzt, dass diese Varianten ein Protein mit einer MIA-2-Aktivität kodieren, oder
b) diese Varianten Nukleinsäureveränderungen aufweisen, die auf die Degenerierung des genetischen Codes zurückzuführen sind, die für dieselbe oder eine funktionell äquivalente Aminosäure wie die Nukleinsäure von SEQ ID NO. 1 kodieren, und
c) die Nukleinsäure nicht ausschließlich aus den Basen 1-354 von SEQ ID NO. 1 oder Bruchstücken hiervon besteht.

Weiterhin betrifft die Erfindung eine murine, isolierte Nukleinsäure, die die Nukleinsäure von SEQ ID NO. 27 oder Varianten hiervon umfasst, wobei die Varianten eine oder mehrere Substitutionen, Insertionen und/oder Deletionen im Vergleich zur Nukleinsäure von SEQ ID NO. 27 aufweisen und für ein Protein kodieren, das durch eine lebespezifische Expression und eine antiproliferative end antifibrotische Wirkung gekennzeichnet ist , vorausgesetzt dass:
a) diese Varianten unter stringenten Bedingungen an eine Nukleinsäure gemäß SEQ ID NO. 27 hybridisieren und weiterhin vorausgesetzt, dass diese Varianten ein Protein mit einer MIA-2-Aktivität kodieren, oder
b) diese Varianten Nukleinsäureveränderungen aufweisen, die auf die Degenerierung des genetischen Codes zurückzuführen sind, die für dieselbe oder eine funktionell äquivalente Aminosäure wie die Nukleinsäure von SEQ ID NO. 27 kodieren, und
c) die Nukleinsäure nicht ausschließlich aus den Basen 1-357 von SEQ ID NO. 27 oder Bruchstücken hiervon besteht.

Die Varianten der erfindungsgemäß bereitgestellten Nukleinsäuren umfassen auch Fragmente, die mehr als 10, bevorzugt mehr als 15, mehr als 20, mehr als 25 oder mehr als 30 und bis zu 50 Nukleotide umfassen. Der Begriff Oligonukleotide umfaßt Fragmente von 10 - 50 Nukleotiden und Teilbereiche hiervon. Diese Sequenzen können beliebig aus den hier vorgestellten Nukleinsäuresequenzen zusammengestellt werden, soweit sie zumindest 10 aufeinanderfolgende Nukleotide hiervon aufweisen. Diese Oligonukleotide können bevorzugt als Primer wie nachstehend ausgeführt eingesetzt werden, z.B. in der RT-PCR.

Ausgehend von den vorstehend offenbarten Nukleinsäuresequenzen kann der Fachmann durch Austesten feststellen, welche Derivate und Abwandlungen, die aus diesen Nukleinsäuren abgeleitet werden können, für spezielle Nachweisverfahren, beispielsweise Hybridisierungsverfahren und PCR-Verfahren, geeignet sind und welche nicht oder weniger geeignet sind. Die Nukleinsäuren und Oligonukleotide der Erfindung können auch beispielsweise in größeren DNA oder RNA-Sequenzen vorliegen, z.B. flankiert von Restriktionsenzymschnittstellen.

Amplifikations- und Nachweisverfahren sind aus dem Stand der Technik bekannt. Nur beispielsweise sei hier auf dem Fachmann bekannte Laborhandbücher verwiesen, die diese Methodik beschreiben, beispielsweise auf Sambrook et al., 1989, Molecular Cloning, A Laboratory Manual, Cold Spring Harbor Press, und alle nachfolgenden Auflagen. PCR-Verfahren sind z.B. beschrieben in Newton, PCR, BIOS Scientific Publishers Limited, 1994 und nachfolgende Auflagen.

Wie oben definiert sind unter "Varianten" erfindungsgemäß insbesondere solche Nukleinsäuren zu verstehen, bei denen eine oder mehrere Substitutionen, Insertionen und/oder Deletionen im Vergleich zu den in den SEQ ID NO. 1 und 27 definierten Nukleinsäuren vorliegen. Bei diesen fehlen vorzugsweise zumindest 1, aber auch 2, 3, 4 oder mehr Nukleotide an einem oder beiden Enden der Nukleinsäuren oder auch im Inneren der Nukleinsäuren oder sind durch andere Nukleotide ersetzt.

Die Nukleinsäuren der vorliegenden Erfindung umfassen also auch Nukleinsäuren, die Sequenzen aufweisen, die im Wesentlichen zu den Nukleinsäuren der SEQ ID No. 1 und 27 äquivalent sind. Erfindungsgemäße Nukleinsäuren können z.B. zumindest ungefähr 80%, typischerweise zumindest ungefähr 90% oder 95% Sequenzidentität zu den Nukleinsäuren der SEQ ID No. 1 und 27 aufweisen.

Der Begriff "Nukleinsäuresequenz" betrifft ein Heteropolymer von Nukleotiden oder die Sequenz dieser Nukleotide. Der Begriff "Nukleinsäure", wie hierin verwendet, umfasst sowohl RNA, DNA, einschließlich cDNA, genomische DNA und synthetische (bsw. chemisch synthetisierte) als auch an andere Polymere gebundene Basen wie PNA.

Die Erfindung umfaßt - wie oben angesprochen - auch solche Varianten, die mit den erfindungsgemäßen Nukleinsäuren unter stringenten Bedingungen hybridisieren.

Unter stringenten Hybridisierungs- und Waschbedingungen versteht man im allgemeinen die Reaktionsbedingungen, unter denen nur noch Duplexmoleküle zwischen Oligonukleotiden und gewünschten Zielmolekülen (perfekte Hybride) entstehen bzw. nur noch der gewünschte Zielorganismus nachgewiesen wird. Unter stringenten Hybridisierungsbedingungen werden dabei insbesondere 0,2 x SSC (0,03 M NaCl, 0,003 M Natriumcitrat, pH 7) bei 65°C verstanden. Bei kürzeren Fragmenten, beispielsweise Oligonukleotiden aus bis zu 20 Nukleotiden, liegt die Hybridisierungstemperatur unter 65°C, beispielsweise bei über 55°C, bevorzugt über 60°C, jeweils aber unter 65°C. Stringente Hybridisierungstemperaturen sind abhängig von der Größe bzw. Länge der Nukleinsäure und ihrer Nukleotidzusammensetzungen und sind vom Fachmann durch Handversuche zu ermitteln. Moderat stringente Bedingungen werden beispielsweise bei 42°C und Waschen in 0,2 x SSC/0,1% SDS bei 42°C erreicht.

Die jeweiligen Temperaturbedingungen können in Abhängigkeit von den gewählten Versuchsbedingungen und in Abhängigkeit von der zu untersuchenden Nukleinsäure-Probe unterschiedlich sein und müssen dann entsprechend angepaßt werden. Der Nachweis des Hybridisierungsprodukts kann beispielsweise durch Autoradiographie im Falle radioaktiv markierter Moleküle oder durch Fluorimetrie bei Verwendung von Fluoreszenz-markierten Molekülen erfolgen.

Der Fachmann kann in an sich bekannter Weise die Bedingungen an das gewählte Untersuchungsverfahren anpassen, um tatsächlich moderat stringente Bedingungen zu erzielen und ein spezifisches Nachweisverfahren zu ermöglichen. Geeignete Stringenzbedingungen lassen sich beispielsweise anhand von Referenzhybridisierungen ermitteln. Eine geeignete Nukleinsäure- bzw. Oligonukleotidkonzentration muß eingesetzt werden. Die Hybridisierung muß bei geeigneter Temperatur stattfinden (je höher die Temperatur, um so schwächer die Bindung der Hybride).

Fragmente der erfindungsgemäßen Nukleinsäuren können z.B. als Oligonukleotidprimer in Nachweisverfahren und Amplifikationsverfahren von MIA-2-Genen und Gentranskripten eingesetzt werden. Der Fachmann kann diese Oligonukleotide in an sich bekannten Verfahren einsetzen. Hierbei wird die DNA oder die RNA einer auf die Anwesenheit der genannten Gene/Gentranskripte zu untersuchenden Probe mit entsprechenden Oligonukleotidprimem in Kontakt gebracht. Anschließend erfolgt der Nachweis der RNA oder der DNA der Probe durch beispielsweise PCR-Techniken, die das Vorliegen spezifischer DNA- und/oder RNA-Sequenzen aufzuzeigen. Alle vorgenannten Oligonukleotide sind auch als Primer für eine reverse Transkription von mRNA einsetzbar.

Das PCR-Verfahren hat den Vorteil, daß sehr kleine DNA-Mengen nachweisbar sind. In Abhängigkeit von dem nachzuweisenden Material sind die Temperaturbedingungen und die Zykluszahlen der PCR abzuwandeln. Die optimalen Reaktionsbedingungen können durch Handversuche in an sich bekannter Weise ermittelt werden.

Die im Verlauf der PCR-Amplifikation durch die Verlängerung der Primersequenzen entstandenen, charakteristischen, spezifischen DNA-Markerfragmente können beispielsweise gel-elektrophoretisch oder fluorimetrisch unter Verwendung fluoreszenzmarkierter Oligonukleotide nachgewiesen werden. Selbstverständlich sind auch andere, dem Fachmann bekannte Nachweisverfahren einsetzbar.

Die DNA oder RNA, insbesondere mRNA, der zu untersuchenden Probe kann sowohl bei der PCR-Reaktion bzw. RT-PCR als auch bei der Hybridisierungsreaktion entweder in extrahierter Form vorliegen oder in Form von komplexen Gemischen, bei denen die zu untersuchende DNA oder RNA nur einen sehr kleinen Anteil an der Fraktion der speziellen biologischen Probe bildet. Die zu untersuchenden Zellen können somit entweder in gereinigter Form vorliegen oder beispielsweise "verunreinigt" im Gewebeverband. Sowohl in situ PCR oder RT-PCR sind mit den hier beschriebenen Oligonukleotiden durchführbar.

Bei der RT-PCR werden die Oligonukleotide der Erfindung zur PCR-Amplifikation von Fragmenten an cDNA Matrizen eingesetzt, die nach einer reversen Transkription von Proben-mRNA erzeugt werden. Die Expression kann dann qualitativ, in Verbindung mit geeigneten Standards und Techniken, wie einem internen Standard und einer quantitativen PCR, auch quantitativ ausgewertet werden.

Gemäß einer Ausführungsform der Erfindung ist die erfindungsgemäße isolierte Nukleinsäure weiterhin mit einer oder mehreren regulatorischen Sequenzen in funktioneller Weise verbunden. Besonders bevorzugt wird hierbei der humane MIA-2-Promotor nach SEQ ID NO. 2 eingesetzt. Ein besonders bevorzugter Teilbereich des Promotors, der noch leberspezifisch ist, umfasst die Basen 2241-3090 von SEQ ID NO. 2.

Die vorliegende Erfindung umfasst weiterhin Transkriptionsprodukte der vorgenannten Nukleinsäuren sowie Nukleinsäuren, die unter stringenten Bedingungen selektiv an eines dieser Transkriptionsprodukte hybridisiert. Es handelt sich hierbei um eine Antisense DNA oder RNA in Form einer DNA- oder RNA-Sonde, die an ein Transkriptionsprodukt, z.B. mRNA, hybridisiert und so zu Nachweiszwecken geeignet ist.

Der Begriff "Sonde", wie er hierin definiert ist, ist durch eine Nukleinsäure definiert, die zur Bindung an eine Zielnukleinsäure einer komplementären Sequenz durch eine oder mehrere Arten chemischer Bindungen in der Lage ist, üblicherweise durch komplementäre Basenpaarung, die üblicherweise durch Wasserstoffbrückenbindung erfolgt.

Zur Detektion weisen die erfindungsgemäßen Nukleinsäuren bevorzugt eine Markierung auf. Beispiele hierfür sind eine radioaktive Markierung, Fluoreszenzmarkierung, Biotinmarkierung, Digoxigeninmarkierung, Peroxidasemarkierung oder eine mit alkalischer Phosphatase nachweisbare Markierung. Die alkalische Phosphatase wird als Markerenzym eingesetzt, da sich in Verbindung mit geeigneten Substanzen ein empfindliches, histochemisches Farbreagenz und Signalverstärkersystem entwickeln lässt. Substrate wie z.B. p-Nitrophenylphosphat ergeben nach der hydrolytischen Spaltung durch das Enzym farbige, photometrisch leicht messbare Reaktionsprodukte.

In einer weiteren Ausführungsform der Erfindung liegen die Nukleinsäuren an eine Festphasenmatrix gebunden vor, z.B. Nylonmembran, Glas oder Kunststoff.

Neben den oben angesprochenen Primem können zur Amplifizierung der humanen Nukleinsäure nach SEQ ID NO. 1, deren Varianten oder der Transkriptionsprodukte hiervon, die forward- bzw reverse-Primer nach SEQ ID NO. 3, 4, 9 oder 26 verwendet werden. Analog können zur Amplifizierung der murinen Nukleinsäure nach SEQ ID NO. 27, deren Varianten oder der Transkriptionsprodukte hiervon, die forward- bzw reverse-Primer nach SEQ ID NO. 3, 7, 9 oder 26 verwendet werden. Zur Amplifizierung des MIA-2 Promotors können eine oder mehrere Nukleinsäuren nach SEQ ID NO. 10-18 verwendet werden.

Gemäß einem weiteren Grundgedanken betrifft die Erfindung humanes MIA-2 Protein, das die Aminosäure von SEQ ID NO. 5 oder eine Variante dieser Aminosäure umfasst, wobei die Varianten eine oder mehrere Substitutionen, Insertionen und/oder Deletionen im Vergleich zur Aminosäure von SEQ ID NO. 5 aufweisen, und wobei die biologische Aktivität des Proteins der Aktivität des MIA-2 Proteins, das die unmodifizierte Sequenz von SEQ ID NO. 5 umfasst, im wesentlichen gleich ist, und durch eine lebespezifische Expression und eine antiproliferative und antifibrotische Wirkung gekennzeichnet ist wobei die Varianten nicht ausschließlich aus den Aminosäureresten 1-119 von SEQ ID NO. 5 bestehen.

Es sei jedoch darauf hingewiesen, dass die vorliegende Erfindung in ihren therapeutischen und diagnostischen Aspekten (s. unten) jeweils auf die Gesamtsequenz der hierin offenbarten Nukleinsäure- und Aminosäuresequenzen oder Teilen hiervon gerichtet ist,
wobei diese Teile u.a. auch die Aminosäurereste 1-119 (human oder murin) bzw. die Nukleinsäure 1-354 (human) und 1-357 (murin) alleine umfassen.

Die Erfindung betrifft weiterhin ein murines MIA-2 Protein, das die Aminosäure von SEQ ID NO. 28 oder eine Variante dieser Aminosäure umfasst, wobei die Varianten eine oder mehrere Substitutionen, Insertionen und/oder Deletionen im Vergleich zur Aminosäure von SEQ ID NO. 28 aufweisen, und wobei die biologische Aktivität des Proteins der Aktivität des MIA-2 Proteins, das die unmodifizierte Sequenz von SEQ ID NO. 28 umfasst, im wesentlichen gleich ist, und durch eine blespezifische Expression und eine antiproliferative und antifibrotische Wirkung gekennzeichnet ist wobei die Varianten nicht ausschließlich aus den Aminosäureresten 1-119 von SEQ ID NO. 28 bestehen.

Insbesondere werden Varianten der Aminosäuresequenz, wie beispielsweise Deletionen, Insertionen und/oder Substitutionen in der Sequenz, die im sich ergebenden Protein-Molekül sogenannte "stille" Veränderungen (silent changes) erzeugen, ebenfalls als innerhalb des Bereichs der vorliegenden Erfindung liegend betrachtet.

Als Beispiel hierfür werden Veränderungen in der Nukleinsäuresequenz betrachtet, die die Erzeugung einer äquivalenten Aminosäure an einer vorgegebenen Stelle zur Folge haben. Vorzugsweise sind derartige Aminosäure-Substitutionen das Ergebnis der Substitution einer Aminosäure durch eine andere Aminosäure mit ähnlichen strukturellen und/oder chemischen Eigenschaften, d.h. konservative Aminosäuresubstitutionen.

Aminosäuresubstitutionen können auf Grundlage der Ähnlichkeit in der Polarität, Ladung, Löslichkeit, Hydrophobie, Hydrophilie, und/oder der amphipatischen (amphiphilen) Natur der involvierten Reste vorgenommen werden. Beispiele für unpolare (hydrophobe) Aminosäuren sind Alanin, Leucin, Isoleucin, Valin, Prolin, Phenylalanin, Tryptophan und Methionin. Polare neutrale Aminosäuren schließen Glycin, Serin, Threonin, Cystein, Thyrosin, Asparagin und Glutamin ein. Positiv geladene (basische) Aminosäuren schließen Arginin, Lysin und Histidin ein. Und negativ geladene (saure) Aminosäuren schließen Asparaginsäure und Glutaminsäure ein.

"Insertionen" oder "Deletionen" bewegen sich typischerweise im Bereich von ein bis fünf Aminosäuren. Der erlaubte Variationsgrad kann experimentell durch systematisch vorgenommene Insertionen, Deletionen oder Substitutionen von Aminosäuren in einem Polypeptidmolekül unter Verwendung von DNA-Rekombinationstechniken und durch Untersuchen der sich ergebenden rekombinanten Varianten bezüglich ihrer biologischen Aktivität ermittelt werden.

Nukleotidveränderungen, die eine Veränderung der N-terminalen und C-terminalen Anteile des Proteinmoleküls zur Folge haben, ändern häufig die Protein-Aktivität nicht, weil diese Anteile üblicherweise nicht in die biologische Aktivität involviert sind. Es kann ebenfalls erwünscht sein, ein oder mehrere der in der Sequenz vorliegenden Cysteine zu eliminieren, weil das Vorhandensein von Cysteinen eine unerwünschte Bildung von Multimeren zur Folge haben kann, wenn die Proteine rekombinant erzeugt werden, wodurch die Reinigung und Kristallisations-Verfahren kompliziert werden. Jede der vorgeschlagenen Modifikationen bewegt sich innerhalb der technischen und naturwissenschaftlichen Grundkenntnisse, genauso wie die Bestimmung der Beibehaltung der biologischen Aktivität der kodierten Produkte.

Gemäß einem weiteren Grundgedanken stellt die vorliegende Erfindung einen Vektor bereit, der eine erfindungsgemäße Nukleinsäure umfasst. Dieser Vektor ist vorzugsweise ein Expressionsvektor, der eine erfindungsgemäße Nukleinsäure und ein oder mehrere regulatorische Sequenzen umfasst.

Zahlreiche Vektoren, die zur Verwendung beim Transformieren von bakteriellen Zellen geeignet sind, sind bekannt, beispielsweise können Plasmide und Bakteriophagen, wie beispielsweise der Phage λ, als die am häufigst verwendeten Vektoren für bakterielle Wirte verwendet werden. Sowohl in Säugetier- als auch Insektenzellen können beispielsweise virale Vektoren zur Expression eines exogenen DNA-Fragments verwendet werden. Beispielhafte Vektoren sind das SV40 oder Polyoma-Virus.

Die Transformierung der Wirtszellen kann alternativ direkt durch "nackte DNA" ohne Verwendung eines Vektors erfolgen.

Die Erzeugung des erfindungsgemäßen Proteins kann entweder in eukaryontischen Zellen oder prokaryontischen Zellen erfolgen. Beispiele von geeigneten eukaryontischen Zellen schließen Säugetierzellen, Pflanzenzellen, Hefezellen und Insektenzellen ein. Geeignete prokaryontische Wirte schließen E.coli und Bacillus subtilis ein.

Bevorzugte Säugetierwirtszelle sind CHO, COS, HeLa, 293T, HEH oder BHK-Zellen oder adulte oder embryonale Stammzellen, wobei menschliche, embryonale Stammwellen ausgenommen sind.

Gemäß einem weiteren Grundgedanken betrifft die vorliegende Erfindung einen Antikörper oder Aptamer, das gegen ein Epitop des erfindungsgemäßen musinen MIA-2 Proteins gerichtet ist.

Der Antikörper ist vorzugsweise aus der Gruppe ausgewählt ist, die aus einem polyklonalen Antikörper, einem monoklonalen Antikörper, einem humanisierten Antikörper, einem chimären Antikörper und einem synthetischen Antikörper besteht.

Der erfindungsgemäße Antikörper kann weiterhin an ein toxisches und/oder nachweisbares Mittel gebunden sein.

Der Begriff "Antikörper", wie er hierin verwendet wird betrifft intakte Antikörper ebenso wie Antikörperfragmente, die eine gewisse Fähigkeit beibehalten, selektiv an ein Epitop zu binden. Derartige Fragmente schließen ohne Einschränkung Fab, F(ab')₂ und Fv Antikörperfragmente ein. Der Begriff "Epitop" betrifft jede Antigendeterminante auf einem Antigen, an die das Paratop eines Antikörpers bindet. Epitop-Determinanten bestehen üblicherweise aus chemisch aktiven Oberflächengruppen von Molekülen (beispielsweise Aminosäure- oder Zuckerreste) und weisen üblicherweise dreidimensionale strukturelle Eigenschaften ebenso wie spezielle Ladungseigenschaften auf.

Die erfindungsgemäßen Antikörper können unter Verwendung irgend eines bekannten Verfahrens hergestellt werden. Beispielsweise kann das reine erfindungsgemäße Protein oder ein Bruchstück hiervon bereit gestellt und als Immunogen verwendet werden, um in einem Tier eine solche Immunreaktion hervorzurufen, dass spezifische Antikörper erzeugt werden.

Die Herstellung polyklonaler Antikörper ist dem Fachmann gut bekannt. Siehe beispielsweise Green et al, Production of Polyclonal Antisera, in Immunochemical Protocols (Manson, Herausgeber), Seiten 1 - 5 (Humana Press 1992) und Coligan et al, Production of Polyclonal Antisera in Rabbits, Rats, Mice and Hamsters, in Current Protocols In Immunology, Abschnitt 2.4.1 (1992). Zusätzlich sind dem Fachmann verschiedene Techniken der Immunologie zur Aufreinigung und Konzentration von polyklonalen Antikörpern bekannt, ebenso wie von monoklonalen Antikörpern (Coligan et al, Unit 9, Current Protocols in Immunology, Wiley Interscience, 1994).

Die Herstellung von monoklonalen Antikörpern ist dem Fachmann ebenfalls vertraut. Siehe beispielsweise Köhler & Milstein, Nature 256: 495 (1975); Coligan et al., Abschnitte 2.5.1 - 2.6.7; und Harlow et al., Antibodies: A Laboratory Manual, Seite 726 (Cold Spring Harbor Pub. 1988). Kurz gesagt können monoklonale Antikörper gewonnen werden, indem Mäusen eine Zusammensetzung die das erfindungsgemäße Protein einschließt injiziert wird, danach das Vorliegen einer Antikörperproduktion durch Untersuchung einer Serumprobe verifiziert wird, die Milz zur Gewinnung von B-Lymphozyten entfernt und die B-Lymphozyten mit Myelomazellen zur Erzeugung von Hybridomas fusioniert werden, die Hybridomas geklont werden, die positiven Klone, die einen monoklonalen Antikörper gegen das Protein erzeugen, selektiert werden und die Antikörper aus den Hybridoma-Kulturen isoliert werden. Monoklonale Antikörper können aus Hybridoma-Kulturen durch eine Vielzahl von wohl etablierten Techniken isoliert und aufgereinigt werden. Derartige Isolierungstechniken schließen eine Affinitätschromatographie mit Protein-A-Sepharose, Größenausschluss-chromatographie und Ionenaustauschchromatographie ein. Siehe beispielsweise Coligan et al., Abschnitte 2.7.1 - 2.7.12 und Abschnitt "Immunglobulin G (IgG)", in Methods In Molecular Biology, Band 10, Seiten 79 - 104 (Humana Press 1992).

Weiterhin stellt die vorliegende Erfindung gemäß einem noch weiteren Grundgedanken eine wie oben beschrieben erzeugte Hybridomazelle bereit, die einen monoklonalen Antikörper produziert, der eine Bindungsspezifität für ein erfindungsgemäßes MIA-2 Protein aufweist.

Die Erfindung umfasst weiterhin eine pharmazeutische Zusammensetzung, die eine erfindungsgemäße Nukleinsäure, einen erfindungsgemäßen Vektor, Protein, Antikörper oder Aptamer als Wirkstoffe in Kombination mit einem pharmazeutisch verträglichen Träger umfasst.

Die Wirkstoffe der vorliegenden Erfindung werden vorzugsweise in Form einer solchen pharmazeutischen Zusammensetzung verwendet, in der sie mit geeigneten Trägern oder Trägerstoffen in Dosen vermischt werden, so daß die Erkrankung behandelt oder zumindest gelindert wird. Eine derartige Zusammensetzung kann (zusätzlich zu den Wirkstoffen und dem Träger) Verdünnungsmittel, Füllmaterialien, Salze, Puffer, Stabilisatoren, Solubilisierungsinittel und andere Materialien enthalten, die in der Technik wohlbekannt sind. Der Begriff "pharmazeutisch verträglich" ist als ein nichttoxisches Material definiert, das die Wirksamkeit der biologischen Aktivität des aktiven Inhaltsstoffes bzw. Wirkstoffes nicht stört. Die Auswahl des Trägers hängt vom Verabreichungsweg ab.

Die pharmazeutische Zusammensetzung kann zusätzlich weitere Mittel enthalten, die die Aktivität des Wirkstoffes steigern oder dessen Aktivität oder Verwendung bei der Behandlung ergänzen. Derartige zusätzliche Faktoren und/oder Mittel können in der pharmazeutischen Zusammensetzung enthalten sein, um eine synergistische Wirkung zu erzielen oder um Nebenwirkungen bzw. unerwünschte Wirkungen zu minimieren.

Techniken zur Formulierung bzw. Zubereitung und Verabreichung der Verbindungen der vorliegenden Anmeldung sind in "Remington's Pharmaceutical Sciences", Mack Publishing Co., Easton, PA, letzte Ausgabe, zu finden. Eine therapeutisch wirksame Dosis bezieht sich ferner auf eine Menge der Verbindung, die ausreicht, um eine Verbesserung der Symptome zu erreichen, beispielsweise eine Behandlung, Heilung, Prävention oder Verbesserung derartiger Zustände. Geeignete Verabreichungswege können beispielsweise orale, rektale, transmucosale oder intestinale Verabreichung und parenterale Verabreichung einschließen, einschließlich intramuskulärer, subkutaner, intramedulärer Injektionen ebenso wie intrathekaler, direkt intraventrikulärer, intravenöser, intraperitonealer oder intranasaler Injektionen. Die intravenöse Verabreichung an einen Patienten wird bevorzugt.

Eine typische Zusammensetzung für eine intravenöse Infusion kann so hergestellt werden, daß sie 250 ml sterile Ringer'sche Lösung und z.B. 10 mg MIA-2 Protein enthält. Siehe Remington's Pharmaceutical Science (15. Ausgabe, Mack Publishing Company, Easton, Ps., 1980).

Die die vorliegenden Wirkstoffe oder eine Mischung hiervon enthaltenden Zusammensetzungen können für prophylaktische und/oder therapeutische Behandlungen verabreicht werden.

Bei einer therapeutischen Anwendung wird die Zusammensetzung einem Patienten verabreicht, der bereits von einer Leberschädigung, z.B. Leberschädigung wie einer Leberzirrhose, Leberfibrose oder einem Lebertumor/-Metastase befallen ist. Die erfindungsgemäßen Nukleinsäuren/Proteine haben sich zur Behandlung der Leberzirrhose und Leberfibrose als hervorragend geeignet erwiesen. Das erfindungsgemäße MIA-2 Gen und das daraus exprimierte MIA-2-Protein weisen insbesondere in der Leber, jedoch auch in anderen Geweben wie Milz und Dünndarm (siehe Tabelle 1) eine ausgezeichnete antiproliferative und antifibrotische Wirksamkeit auf. Für ausführliche Informationen wird auf den experimentellen Teil der Anmeldung verwiesen.

Eine Menge, die adäquat ist, um die vorgenannten Wirkungen zu erreichen, wird als eine "therapeutisch wirksame Dosis" definiert. Mengen, die für diese Verwendung wirksam sind, hängen von der Schweregrad des Zustandes und dem Allgemeinzustand des patienteneigenen Immunsystems ab, bewegen sich jedoch im allgemeinen von ungefähr 0,01 bis 100 mg Protein pro Dosis, wobei Dosierungen von 0,1 bis 50 mg und von 1 bis 10 mg pro Patient üblicherweise verwendet werden. Einfache oder mehrfache Verabreichungen nach einem täglichen, wöchentlich oder monatlichen Behandlungsplan können mit Dosisstärken und Mustern durchgeführt werden, die durch den behandelnden Arzt ausgewählt werden.

Eine pharmazeutische Zusammensetzung, die ein erfindungsgemäßes MIA-2 Protein in Kombination mit einem pharmazeutisch verträglichen Träger umfasst, kann wahlweise weitere Wirkstoffe wie z.B. Interferone, Inhibitoren des ACE-Systems oder Liganden des proliferation-activated receptor-g (PPAR-g) aufweisen, die die antifibrotische Wirksamkeit des MIA-2 Proteins weiter fördern.

Gemäß einem weiteren Grundgedanken umfasst die Erfindung weiterhin eine diagnostische Zusammensetzung, die einen erfindungsgemäßen Antikörper oder Aptamer umfasst.

Die Erfindung betrifft weiterhin ein transgenes, nicht menschliches Säugetier, bei dem eine oder mehrere der erfindungsgemäßen MIA-2-Sequenzen inaktiviert wurde. Unter Verwendung der Technologie der homologen Rekombination kann ein knock-out-Tiermodell etabliert werden, das weitere Einsichten in die Ätiologie von Leberschädigungen etc. bereitstellt.

Vorzugsweise umfasst die Erfindung eine transgene Maus, bei der eine erfindungsgemäße Nukleinsäure konditionell inaktiviert wurde. Dies stellt einen Spezialfall innerhalb der Knock-Out-Technologie dar. Die ursprünglichen Anwendungen der Knock-Out-Technologie resultierten nämlich grundsätzlich in einer konstitutiven Ausschaltung des zu untersuchenden Gens. Vorzugsweise wird deshalb in der vorliegenden Erfindung ein Zelltyp-spezifisches und zeitlich regulierbares System verwendet, das eine konditionelle Ausschaltung eines Gens in einem bestimmten Gewebe oder Zelltyp zu einem bestimmten Zeitpunkt ermöglicht. Für die konditionelle Genausschaltung wird zum einen ein spezifischer Promoter gebraucht, der das gewünschte Gen nur in den selektierten Zellen ausschaltet. Dazu wird z.B. der MIA-2 Promoter mit einer Rekombinase, z. B. Cre auf der DNA Ebene fusioniert. Dieses Konstrukt wird zur Herstellung transgener Säugetiere verwendet, die folglich diese Rekombinase spezifisch in der Leber exprimieren. Die Gene, die gewebe-spezisch ausgeknockt werden sollen, werden so kloniert, dass sie von spezifischen Erkennungssequenzen für die Rekombinase, im Falle von Cre von loxP Sequenzen, flankiert und werden dann mittels der Knock-Out-Technologie in ES Zellen transferiert. Die daraus resultierenden Säugetiere werden mit den Rekombinaseexprimierenden Säugetieren gekreuzt, was dann in der gewebe-spezifischen Deletion der zu analysierenden Gene führt. Bei Verwendung des MIA-2 Promotors können die zu untersuchenden Gene spezifisch in der Leber inaktiviert werden.

Weiterhin umfasst die vorliegende Erfindung ein transgenes Säugetier, in dessen Genom eine erfindungsgemäße Nukleinsäure eingefügt wurde. Beispielsweise kann der erfindungsgemäße Promotor mit weiteren cDNA's kombiniert werden. Mittels des MIA-2 Promoters können andere cDNAs, auch solche, die natürlicherweise nicht in der Leber vorkommen, spezifisch in der Leber überexprimiert werden und auf ihre Funktion hin untersucht werden. Diese Methode kann zur Targetvalidierung und Identifizierung potentieller neuer Therapeutika für die Leber verwendet werden.

Gemäß einem weiteren Grundgedanken betrifft die vorliegende Erfindung ein ex vivo-Verfahren zur Diagnose einer Leberschädigung oder zur Bestimmung der hepatischen Syntheseleistung das folgende Schritte umfasst:
a) Bereitstellung einer Lebergewebe- oder Serumprobe oder Biopsie von einem Patienten,
b) Qualitative und/oder quantitative Bestimmung der wie oben definierten Transkriptionsprodukte in der Probe, oder der Transkriptionsprodukte einer Nukleinsäure, die die Nukleinsäure von SEQ ID NO. 1 oder 27 oder Varianten hiervon umfasst, wobei die Varianten eine oder mehrere Substitutionen, Insertionen und/oder Deletionen im Vergleich zur Nukleinsäure von SEQ ID NO. 1 oder 27 aufweisen, vorausgesetzt dass:
   - diese Varianten unter stringenten Bedingungen an eine Nukleinsäure hybridisieren, die die Sequenz von SEQ ID NO. 1 oder 27 umfasst und weiterhin vorausgesetzt, dass diese Varianten ein Protein mit einer MIA-2-Aktivität kodieren, oder
   - diese Varianten Nukleinsäureveränderungen aufweisen, die auf die Degenerierung des genetischen Codes zurückzuführen sind, die für dieselbe oder eine funktionell äquivalente Aminosäure wie die Nukleinsäure von SEQ ID NO. 1 oder 27 kodieren,
wobei eine Überexpression der Transkriptionsprodukte auf eine Leberschädigung und/oder eine gesteigerte hepatische Syntheseleistung hinweist.

Vorzugsweise erfolgt die Bestimmung in Schritt b) durch Northern Blot, in situ Hybridisierung oder RT-PCR oder einer Kombination hiervon. Für weitere Informationen siehe auch McPherson et al. (Hrsg.), PCR, A Practical Approach, Oxford, IRL Press 1995.

Eine RT-PCR wird vorzugsweise mit den Primem der SEQ ID NO. 3 und 9 (humanes MIA-2) bzw. 3 und 7 (murines MIA-2) durchgeführt.

Weiterhin kann eine Bestimmung von MIA-2 unter Verwendung einer oben beschriebenen diagnostischen Zusammensetzung erfolgen, die anti-MIA-2 Antikörper oder -Aptamere oder MIA-2 spezifische DNA oder RNA-Sonden aufweist. Dies wird im Folgenden dargelegt:

Ein erfindungsgemäßes ex vivo-Verfahren zur Diagnose einer Leberschädigung, oder zur Bestimmung der hepatischen Syntheseleistung, umfasst die folgenden Schritte:
a) Bereitstellung einer Lebergewebe- oder Serumprobe oder Biopsie von einem Patienten,
b) in Berührung bringen der Probe mit einem Reagenz, das zum Nachweis des MIA-2-Proteins nach SEQ ID NO: 5 oder 28 oder einer Variante dieser Aminosäure in der Lage ist, wobei die Variante eine oder mehrere Substitutionen, Insertionen und/oder Deletionen im Vergleich zur Aminosäure von SEQ ID NO. 5 oder 28 aufweist, und wobei die biologische Aktivität des Proteins der Aktivität des MIA-2 Proteins, das die unmodifizierte Sequenz von SEQ ID NO. 5 oder 28 umfasst, im wesentlichen gleich ist und durch eine lebespezifische Expression und eine antiproliferative und antifibrotische Wirkung gekennzeichnet ist,
c) Qualitative und/oder quantitative Bestimmung des MIA-2-Proteins in der Probe, wobei das Vorliegen und die Menge des MIA-2-Proteins auf eine Leberschädigung und/oder eine gesteigerte hepatische Syntheseleistung hinweist.

Gemäß einer bevorzugten Ausführungsform ist das Reagenz ein Antikörper, der gegen ein Epitop des MIA-2-Proteins gerichtet ist.

Dieser Antikörper ist vorzugsweise aus der Gruppe ausgewählt, die aus einem polyklonalen Antikörper, einem monoklonalen Antikörper, einem humanisierten Antikörper, einem chimären Antikörper und einem synthetischen Antikörper besteht, und ist vorzugsweise an ein nachweisbares Mittel gebunden.

Insbesondere können die oben genannten Diagnoseverfahren bei einer potentiellen Leberschädigung wie einer Leberzirrhose, Leberfibrose oder einem Lebertumor/-Metastase eingesetzt werden.

Die erfindungsgemäßen pharmazeutischen Zusammensetzung finden insbesondere in der antifibrotischen Therapie Anwendung, s.o., insbesondere jedoch in der Behandlung der Leberzirrhose oder Leberfibrose bzw. in der Behandlung von Lebertumoren/-Metastasen.

Ein Verfahren zur Herstellung einer Organkultur, kann die folgenden Schritte umfassen:
a) Bereitstellung von Säugetierhepatozyten in einem Wachstumsmedium
b) in Berührung Bringen der Säugetierhepatozyten mit einem MIA-2 Protein nach wie oben definiert
c) Isolierung der erzeugten Organkultur.

In Schritt a) werden insbesondere humane oder Schweine-Hepatozyten eingesetzt.

Die dadurch hergestellte Organkultur kann vorteilhaft zur ex vivo Blutreinigung von Patienten, die aufgrund einer Leberschädigung eine nicht ausreichende Leberfunktion aufweisen, eingesetzt werden.

Die vorliegende Erfindung wird nachfolgend anhand mehrerer Abbildungen und den begleitenden Beispielen beschrieben, die jedoch den Umfang der Erfindung nicht einschränken sollen, sondern diesen lediglich veranschaulichen.

Abb. 1 zeigt einen Vergleich der humanen MIA, OTOR, MIA-2 und TANGO cDNA-Sequenzen.
(a) Sequenzalignment der vier menschlichen homologen MIA cDNA-Sequenzen.
(b) Homologie zwischen den Mitgliedern der MIA Genfamilie auf cDNA Ebene, dargestellt als Stammbaum. Der Baum wurde unter Verwendung des Programmes DNAman auf Grundlage des alignments der vollständigen kodierenden Region konstruiert. Die Länge jeder horizontalen Linie ist dem Grad der cDNA-Sequenzdivergenz proportional.

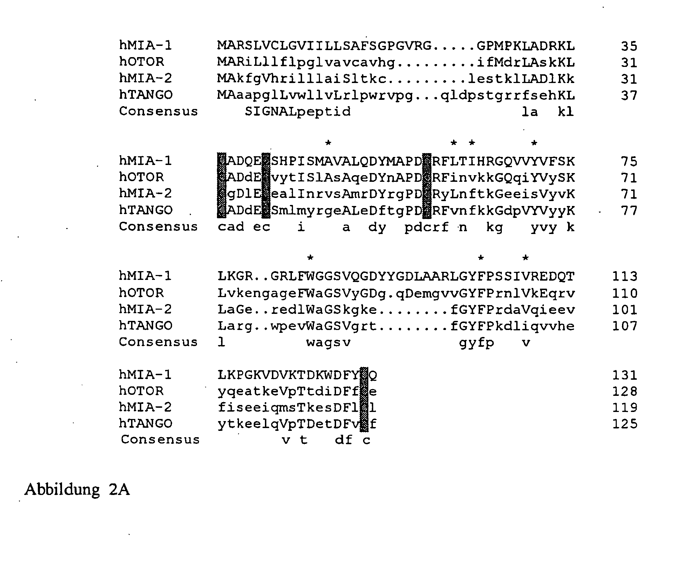

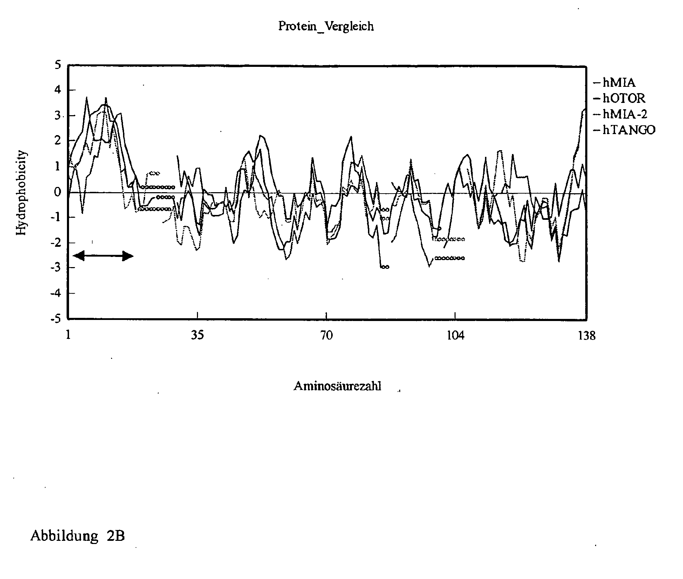

Abb. 2 zeigt einen Vergleich der menschlichen MIA, OTOR, MIA-2 und TANGO Peptidsequenzen
(a) Sequenzalignment der vier humanen homologen MIA Aminosäuresequenzen. Konservierte Cystinreste sind mit einer Box markiert. Die mit einem Stern (*) markierten Reste sind für den hydrophoben Kern der SH3 Domäne von Bedeutung.
(b) Kyte-Dolittle-Blot, der die Hydrophobie der homologen MIA-Proteine analysiert. Der Pfeile zeigt hochhydrophobe Signalsequenzen an.

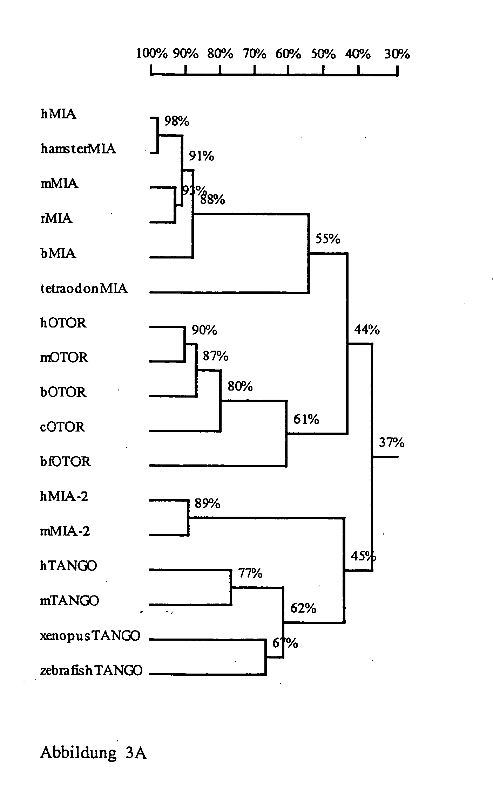

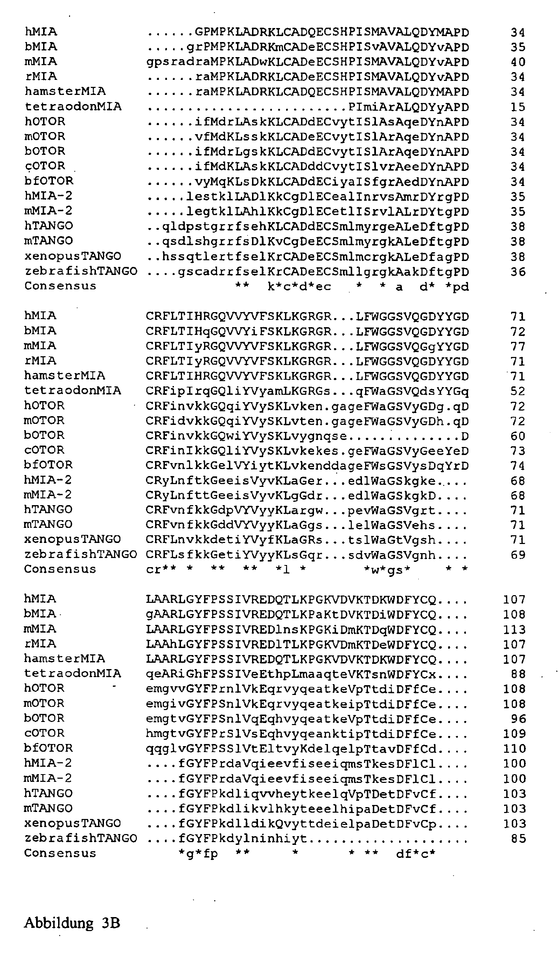

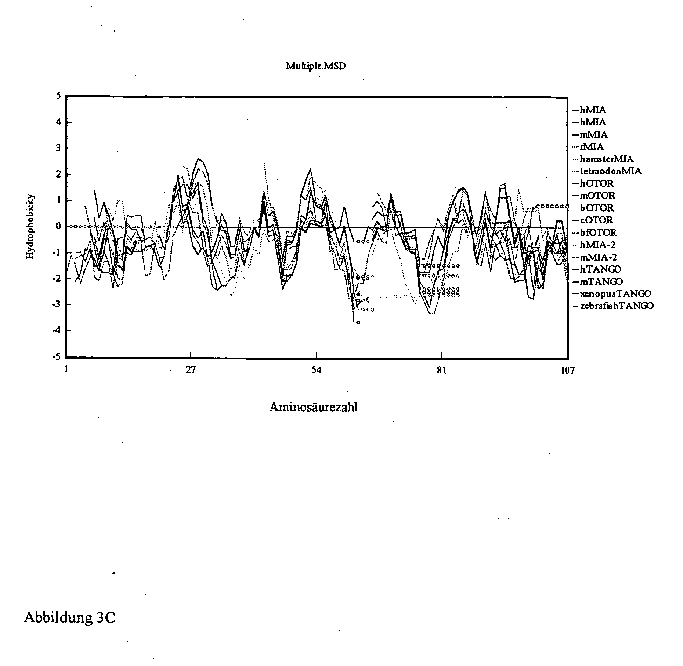

Abb. 3 zeigt einen Vergleich aller verfügbaren Sequenzen der MIA Gen-Familie
(a) Die Homologie zwischen allen MIA Gen-Familienmitgliedern auf der Proteinebene ist als Stammbaum dargestellt. Die Spezies sind wie folgt abgekürzt: h = human, b = bovin, m = murin, r = Ratte, bf = Ochsenfrosch, c = Huhn. Der Baum wurde unter Verwendung des Programms DNAman auf Grundlage des Alignments der abgeleiteten Aminosäure-sequenzen konstruiert. Um die nachteilhaften Auswirkungen der variablen N-terminalen Signalpeptide auszuschließen wurden nur die Sequenzen der reifen Proteine verglichen. Tetraodin-MIA und Zebrafisch-TANGO sind nur als Partial-Klone verfügbar.
(b) Aminosäurevergleich aller verfügbaren Familienmitglieder des MIA-Gens. Die Aminosäuresequenzen sind im Single-Letter-Code angegeben, die Zahlen zeigen die Reste bezüglich der initialen Aminosäure des reifen Proteins ohne der Signalsequenz an. Identische Reste sind in der unteren Zeile angezeigt; Ähnlichkeiten sind durch * gekennzeichnet.
(c) Ein Kyte-Dolittle-Blot zeigt die hochkonservierte Gesamtstruktur in unterschiedlichen Spezies.

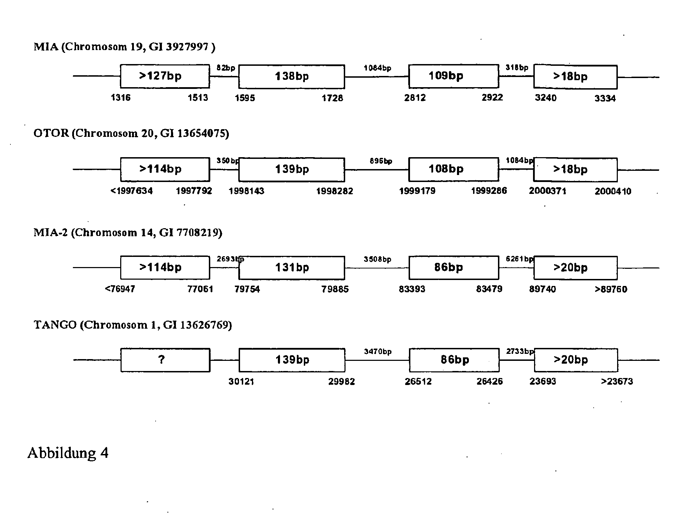

Abb. 4 zeigt die genomische Organisation der humanen MIA Genfamilie
Die Exon-Intronstruktur wurde durch Anpassen der cDNA-Sequenz an die äquivalente genomische Region konstruiert. Exons und Introns sind jeweils durch Boxen bzw. Linien dargestellt. Die Anzahl der Boxen zeigt die Länge jedes Exons an. Die humane genomische TANGO-Sequenz ist noch unvollständig.

Abb. 5 zeigt die RNA in situ-Hybridisierung auf Schnitten von Mausembryonen (Embryonalstadium-Tag 12,5 und 14,5).

Abb. 6 zeigt den Einfluss von MIA-2 auf die Proliferation von aktivierten HSZs.

Abb. 7 zeigt den Nachweis der Expression von MIA-2 mittels RT-PCR in verschiedenen humanen und murinen Geweben.

Abb. 8 zeigt die Expression von MIA 2 in primären humanen Hepatozyten.

Abb. 9: in Hepatitis Patienten mit milder Fibrose wird signifikant weniger MIA-2 exprimiert als in Hepatitis Patienten mit fortgeschrittener Fibrose.

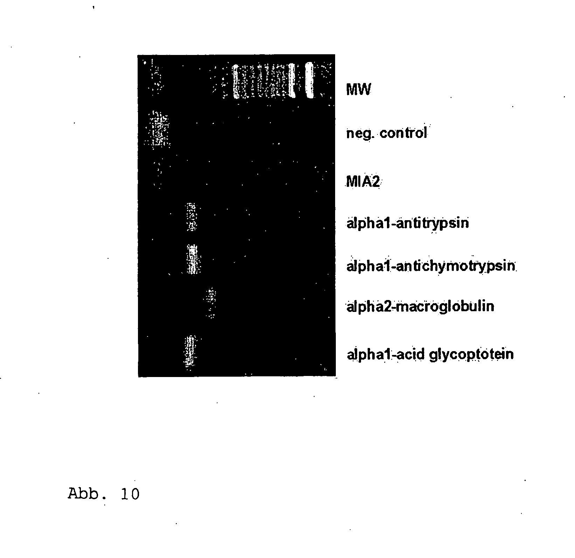

Abb.10: RNA wurde von primären humanen Hepatozyten isoliert und die mRNA Expression wurde für MIA2, alpha-1-Antitrypsin (alpha1-AT), alpha 2-Makrogobulin (alpha2-MG), alpha 1-acid glykoprotein (alpha1-AG) und alpha 1-Antichymotrypsin (alpha1-ACT) mittels semi-quantitativer PCR analysiert. Für die RT-PCR Analyse wurden jeweils 1 µl von der gleichen cDNA Präparation eingesetzt. Die PCR lief über 28 Zyklen.

### Primersequenzen:

alpha1-AT forw: GGG AGA GAC CCT TTG AAG TCA (SEQ ID NO: 29)
and alpha1-AT rev: AAG AAG ATG GCG GTG GCA T; (SEQ ID NO: 30)

alpha2-MG forw: CAG TGG AGA AGG AAC AAG CG (SEQ ID NO: 31)
alpha2-MG rev: TTG GTG GCA GTT TCA GGG ATA; (SEQ ID NO: 32)

alpha1-AG forw: ACA CCA CCT ACC TGA ATG TCC (SEQ ID NO: 33)
and alpha1-AG rev: ACT CTC CCA GTT GCT CCT TG; (SEQ ID NO: 34)

alpha1-ACT forw: GCC CAT AAT ACC ACC CTG ACA (SEQ ID NO: 35)
and alphal-ACT rev: TAC AGC CTC TTG GCA TCC TC. (SEQ ID NO: 36)

Im Allgemeinen können die erfindungsgemäßen Therapieansätze wie folgt beschrieben werden:

### a) Fibrosemarker/ Parameter für Leberschädigung

Sowohl für die Therapie, als auch für die Vorhersage des Verlaufes von Lebererkrankungen und damit u.a. auch für Screening- und Vorsorgeuntersuchungen ist es von entscheidender Bedeutung das Ausmaß der Lebererkrankung zu kennen. Entscheidende Größen der hepatischen Gewebsschädigung sind das Ausmaß der Entzündung und das Ausmaß der Fibrosierung. Goldstandard und letztendlich auch einziger derzeit existierender, zuverlässiger Parameter für diese Fragestellungen ist die histologische Untersuchung von z.B. durch Biopsie entnommenem Lebergewebe. Wünschenswert, da für den Patienten wesentlich weniger belastend wäre die Analyse entsprechender Serumparameter als zuverlässige Parameter für das Ausmaß der hepatischen Entzündung und Fibrose. Auch für Verlaufsuntersuchungen, z.B. zur Kontrolle des Therapieansprechens, wären solche Parameter von entscheidender Bedeutung, da hier eine wiederholte engmaschige Gewebeentnahme nicht möglich ist.

Fibroseparameter mit hinreichender Sensitivität und Spezifität, die einen Einsatz in der Klinik ermöglichen und rechtfertigen existieren derzeit nicht.

Serum-Transaminasen reflektieren z.B. bei chronisch viralen Lebererkrankungen nur unzureichend oder bei vielen Patienten auch gar nicht das Ausmaß der hepatischen Entzündungsreaktion.

Die Fibrosierungsreaktion als Korrelat für eine "Vernarbung" nach Gewebsschädigung oder - reizung verläuft generell in den meisten Geweben und als Reaktion auf unterschiedliche Noxen relativ uniform. So kommt es beispielsweise auch in den Nieren, der Lunge, im Darm oder in der Haut infolge chronischer Entzündung zur Fibrosierung. Auch für diese Erkrankungen bzw. Organsysteme gilt ähnliches wie für Lebererkrankungen: 1) Kenntnis über das Ausmaß der Fibrose ist wichtig für Behandlungs- und Präventions-Strategien 2) serologische Parameter hierfür wären hilfreich, existieren jedoch nicht in geeigneter Form.

### b) Tumormarker

Eine der schwerwiegendsten Komplikationen v.a. fortgeschrittener Lebererkrankungen ist die Entstehung des hepatozellulären Karzinoms (HCC), das sehr häufig zum Tod führt (4.häufigste Krebstodesursache). Auch extrahepatische Tumoren metastasieren häufig in die Leber. Das Screening nach solchen Tumoren oder Metastasen erfolgt derzeit suffizient nur durch entsprechende Bildgebung. Die Sicherung der Diagnose erfolgt ausschließlich durch Biopsie und histologische Untersuchung der entsprechenden Prozesse. Es wäre wünschenswert, wenn sowohl beim Screening, als auch bei der Diagnose zuverlässige Serumparameter zur Verfügung stehen würden. Für Metastasen extrahepatischer Tumoren gibt es solche nicht, für hepatische Tumoren (hepatozelluläres Karzinom HCC) gibt es z.B. mit dem AFP (alpha-feto-Protein) nur Parameter mit unzureichender Sensitivität und Spezifität (Lun-Xiu Qin, Zhao-You Tang, World J Gastroenterol 2002;8(3):385-392; Matsumura M et al.. J Hepatol 1999;31:332-339). Die nunmehr durch die vorliegende Erfindung bereitgestellte MIA-2 Sequenz kann daher als herrvorragender Tumormarker dienen.

### c) antifibrotische Therapie

Einzige wirksame antifibrotische Therapie chronischer Lebererkrankungen ist derzeit die Unterbrechung der pathophysiologischen Ursache der Erkrankung. Gesicherte Therapieformen das Fortschreiten der hepatischen Fibrosierung bei persistierender Noxe aufzuhalten oder gar eine bereits manifeste Fibrose oder Zirrhose der Leber wieder rückgängig zu machen existieren nicht.

Wie unter a) beschrieben gilt eine Analogie auch für andere Organsysteme als die Leber. Gute antifibrotische therapeutische Strategien fehlen auch hier und potentielle therapeutische Strategien für Lebererkrankungen könnten auch bei anderen Organsystemen eingesetzt werden.

Wie dargestellt gibt es derzeit für die genannten Anwendungsgebiete
a) Fibrosemarker
b) Marker für hepatische Schädigung/Syntheseleistung
c) Marker für hepatische Tumoren und hepatische Metastasen extrahepatischer Tumoren
d) antifibrotische Therapie
praktisch keine hinreichenden Lösungsmöglichkeiten, obwohl jene in der Klinik dringend erforderlich wären. Auch hier eröffnet MIA-2 nunmehr zahlreiche neue Ansätze.

Im Tierversuch zeigen sich dabei vereinzelt Erfolge und einige Pharmaka und Diagnostika werden im Rahmen klinischer Studien auch erprobt. Wie oben beschrieben gibt es derzeit jedoch noch keine gesicherte Therapieformen oder zuverlässige diagnostische Parameter.

### Beispiele

### Beispiel 1: Klonierung von MIA-2

### Beispiel 1a : Klonierung der MIA-2 cDNA, die für das MIA-2 Protein kodiert

Zur Amplifikation der MIA2 cDNA wird eine RT-PCR mit spezifischen Primern (SEQ ID NO 3 und SEQ ID NO 4 bzw. SEQ ID NO 9 für die humane Sequenz, sowie SEQ ID NO 3 und SEQ ID NO 7 bzw. SEQ ID NO 9 im Falle der murinen Sequenz) durchgeführt. Hierzu wird RNA aus humanem bzw. murinen Lebergewebe isoliert, und dann mit Hilfe einer reversen Transkription in cDNA umgeschrieben. Anschließend wird diese cDNA in einer PCR Reaktion mit den entsprechenden MIA2 Oligonucleotidprimem (s.o.) eingesetzt. Das PCR Produkt wird über eine blunt-end-Klonierung in den Vektor pPCR-Script (Stratagene, Katalog Nr. 211188) kloniert.

### Beispiel 1b: Klonierung des MIA-2 Promoters

Mittels spezifischer PCR Primer wurde der MIA-2 Promoter mit genomischer DNA als Template amplifiziert und in die Bgl II und Hind III Restriktionsschnittstellen in den pGL3-basic Vektor (Promega) kloniert. Für die PCR Amplifizierung wurden folgende SEQ ID NO 10 bis SEQ ID NO 17 als "forward" primer und SEQ ID NO 18 als "reverse primer" eingesetzt.

### Beispiel 2.: Rekombinante Expression von humanem MIA-2 in vitro

Zur in vitro Translation wird die MIA-2 cDNA bzw. Mutanten des MIA-2, die für bestimmte Anwendungen besser geeignet sind (stabiler, höhere Affinität zum Substrat ), in ein eukaryontisches Expressionplasmid System kloniert. Die Vektoren verfügen, neben den zur Amplifikation und Erhalt in E.coli notwendigen Motiven, über einen T7-Promoter und eine T7-Termination-Sequenz, ferner über geeignete Schnittstellen zur Klonierung der MIA-2 cDNA (z.B. pIVEX2.3-MCS, Roche). Im Falle des pIVEX2.3-MCS wurde MIA2 mittels der Primer SEQ ID NO 19 und SEQ ID NO 9 amplifiziert und anschließend über NdeI und Bam HI in den Vektor kloniert. Mit kommerziell erwerbbaren in-vitro-Translationssystemen (RTS-System, Roche; ECL cell in vitro translation system, Amersham Pharmacia Biotech; PROTEINscript-PRO, Ambion) wird mittels der klonierten Expressionsplasmide rekombinantes MIA-2 Protein hergestellt. Der Nachweis der Expression erfolgt über spezifische Antikörper im Western Blot oder mit ELISA.

### Beispiel 3: Rekombinante Expression von humanem MIA-2 in eukaryontischen Zellen

### Beispiel 3a: Rekombinante Expression von MIA-2 in Säugetierzellen

Für die Expression von MIA-2 in Säugetierzellen wird die cDNA für MIA-2, bevorzugterweise humanes MIA-2 (SEQ ID NO 1 oder SEQ ID 20), in einen geeigneten Expressionsvektor kloniert. Dieser Expressionsvektor wird in Säugetierzellen transkribiert und hat ein effizientes Promoter-Enhancer System, um ausreichende MIA-2 Proteinmengen zu produzieren (im Falle, dass genomische MIA-2 DNA Fragmente verwendet werden, ist dieses notwendig, da MIA-2 nur in bestimmten Zelltypen aktiv ist, e.g. Hepatozyten, und nicht ein ubiquitäre Expression ermöglicht; jedoch kann eine ubiquitäre Expression mittels homologer Expression in vitro hergestellt werden). Solche Promoter und Enhancer sind häufig von Viren wie z. B. SV40, hCMV, polyoma oder Retroviren isoliert. Alternativ können auch Promoter-Enhancer Systeme verwendet werden, die gewebespezifisch, z. B: WAP-, MMTV- oder Immunglobulinpromoter oder induzierbar sind, wie z. B. der Metallothionein-promoter. Der Expressionsvektor stellt der MIA-2 cDNA (falls diese verwendet wird), Splice Akzeptor und Donor Signale für die RNA Prozessierung und einen polyA Schwanz zur Verfügung. Z. B. pCDNA3 (Invitrogen, San Diego, USA), ist ein geeigneter Expressionsvektor. In die einzige EcoRI Restriktionsseite wird die MIA-2 cDNA nach Anfügen von EcoRI Linkem kloniert, wobei durch weitere Restriktionsanalysen mit geeigneten Restriktionsenzymen überprüft wird, dass MIA-2 in korrekter Orientierung in den Expressionsvektor kloniert wurde. Eine analoge Prozedur wird für andere Expressionsvektoren verwendet, wie z. B. pCMX-pL1 (Umesono et al., Cell 65 (1991) 1255-1266), oder pSG5 (Stratagene, LaJolla, USA). Im Falle des pCMX-MIA2 Expressionvektors wurde MIA2 mittels der Primer SEQ ID NO 18 und SEQ ID NO 19 amplifiziert und anschließend über EcoRI oder blunt end Ligation in den Vektor kloniert.

Die DNA des so erhaltenen Expressionsplasmid wird aus E. coli präpariert. Anschließend werden die Säugetierzellen transfektiert und selektiert, mit der entsprechenden für die jeweilige Zelllinie optimal Methode. (Methods of Enzymology 186 (Gene Expression Technology), ed. David V. Goeddel, Academic Press 1991, Sektion V). Als Säugetierzellen können CHO, COS, 3T3, MelIm Zellen verwendet werden. Bei der Expression von MIA-2 konnte MIA-2 Protein im Zellkulturüberstand nachgewiesen werden.

### Beispiel 3b: Rekombinante Expression von MIA-2 in Insektenzellen

Für die Expression von MIA-2 in Insektenzellen wird die cDNA für MIA-2, bevorzugterweise humanem MIA-2 (SEQ ID NO 20 oder SEQ ID NO 1), in einem geeigneten Expressionsvektor, der sich von AcMNPV (Autographa californica multicapsid nucleopolyhedrosis virus) oder BmNPV (*Bombyx mori* nucleopolyhedrovirus) herleiten läßt, kloniert. Zuerst wird die MIA-2 cDNA so kloniert, dass MIA-2 von einem starken Promoter, der in Insektenzellen aktiv ist, reguliert wird. Solch ein Promoter wäre polH (polyhedrin) oder p10 (D. R. O'Reilly, L. K. Miller und V. A. Luckow, Baculovirus expression Vectors - A Laboratory Manual (1992), W. H. Freeman & Co., New York). Um MIA-2 mit der Hilfe von polH zu exprimieren, wird folgendes durchgeführt: Ein cDNA fragment, das für MIA-2 kodiert (SEQ ID NO 20 oder SEQ ID NO 1), wird in einen Transfervektor, z. B: pVL1393 (D. R. O'Reilly, L. K. Miller und V. A. Luckow, Baculovirus expression Vectors - A Laboratory Manual (1992), W. H. Freeman & Co., New York) mittels blunt end Ligation kloniert. Dieser Tranfervektor ist kommerziell erhältlich (BD Pharmingen, San Diego, California oder Invitrogen Corporation, San Diego, California). Das so erhaltene Transferplasmid pVL1393-MIA-2 wird zur Vermehrung in E. coli K12 transfiziert und Plasmid DNA wird nach üblichen Methoden isoliert (Sambrook et al., 1989, Molecular Cloning, A Laboratory Manual, Cold Spring Harbor Press, A Laboratory Manual, Cold Spring Harbor Press, und alle nachfolgenden Auflagen). Der Transfer der MIA-2 DNA vom Transferplasmid in den Baculovirus Vektor erfolgt mittels homologer Rekombination nach etablierten Methoden (D. R. O'Reilly, L. K. Miller und V. A. Luckow, Baculovirus expression Vectors - A Laboratory Manual (1992), W. H. Freeman & Co., New York). Dafür werden 0,5 µg BD BaculoGold™ DNA (linearisierter, modifizierte AcNPV Baculovirus DNA mit einer lethalen Deletion und lacZ Expression, die vom polH Promoter kontrolliert wird, ist von BD Pharmingen kommerziell erhältlich, Bestellnr. 21484P) und 2 µg pVL1393-MIA-2 werden gemischt, bei Raumtemperatur für 5 Minuten inkubiert und anschließend mit 1 ml einer Lösung aus 125 mM Hepes pH 7,1, 125 mM CaCl₂ und 140 mM NaCl gemischt. Diese Mischung wird auf 2x10⁶ SF9 Insektenzellen (Invitrogen, Bestellnr. B825-01 oder BD Pharmingen Bestellnr. 551407) in einer Zellkuturschale mit einem Durchmesser von 60 mm, die zuvor mit 1 ml Grace's Medium plus 10% FBS (foetals Rinderserum) beschichtet wurde, gegeben. Nach 4 Stunden Inkubation bei 4 °C wird das Medium, das die DNA enthält, entfernt und die Zellen werden in frischen Medium bei 27 °C für 4 Tage kultiviert. Die so erhaltenen rekombinanten Baculoviren werden zweimal mittles Plaque Bildung gereinigt (D. R. O'Reilly, L. K. Miller und V. A. Luckow, Baculovirus expression Vectors - A Laboratory Manual (1992), W. H. Freeman & Co., New York). Die rekombinanten Baculoviren, die MIA-2 enthalten, können dadurch unterschieden werden, dass sie keine lacZ Expression und somit keine β-Galaktosidase Aktivität haben. Mit den so erhaltenen MIA-2 - exprimierenden rekombinanten Baculovirus werden SF9 Zellen nach den üblichen Methoden (D. R. O'Reilly, L. K. Miller und V. A. Luckow, Baculovirus expression Vectors - A Laboratory Manual (1992), W. H. Freeman & Co., New York) infiziert (MOI = 20 pfu/Zelle).

Die Zellen werden für mindestens 36 Stunden bei 27 °C in serum-freien Medium kultiviert (z. B. BD BaculoGold Max-XP Insect Cell Medium, BD Pharmingen, Bestellnr. 551411). Anschließend werden die Zellüberstände entfernt, der Virus wird vom Überstand durch Ultrazentrifugation (Beckmann Ti 60 Rotor, 30,000 rpm) getrennt. Danach wird der Überstand durch Microcon 100 Filter (Amicon, Ausschlußgrenze von 100 kD) gefiltert. Die so erhätliche MIA-2 Lösung kann entweder direkt für Testzwecke verwendet werden oder weiter aufgereinigt werden.

### Beispiel 4: Rekombinante Expression von humanem fusionsfreiem MIA-2 in Escherichia coli

Klonierung der MIA-2 cDNA in einen *E. coli*-Expressionsvektor Zur Klonierung der MIA-2 cDNA wurde eine geeignetes Expressionssystem, wie z. B. das T7-Expressionssystem der Firma Novagen (Studier und Moffat, J. Mol. Bio. 189 (1986), 113-130) oder Vektorsystem pQE40, pGST oder ähnlichen verwendet (z. B. Firma Qiagen, Cat. No. 33403). Als Ausgangs-DNA diente der pBS-Vektor mit inserierter MIA-2 -cDNA (SEQ ID NO 20 bzw. SEQ ID NO 1). Die MIA-2 -cDNA wurde so verändert, dass eine effiziente Expression im E. coli System ermöglicht wird. Die Primer zur Amplifikation von MIA-2 enthielten Schnittstellen, die eine Insertion der cDNA in geeignete Vektorsysteme ermöglicht. Nach den Amplifikation wird das PCR Produkt mit den jeweiligen Restriktionsenzymen geschnitten und so die Klonierung in den vorbereiteten Vektor (gespalten und vor Re-Ligation geschützt durch Dephosphorylierung) ermöglicht. Nach der Ligation wird das Konstrukt aus MIA-2-Insert und Vektor in E. coli transformiert und das korrekte Konstrukt isoliert. Die Nukleotidsequenz wurde nach Abschluss der Klonierung mittels DNA-Sequenzierung (Sanger et al. PNAS 74 (1977), 5463-67) mit MIA-2 spezifischen Primem (SEQ ID NO. 21 und 22) überprüft.

Das Expressionsplasmid wird in einen geeignetem *E. coli* Stamm, wie z. B. BL21 (DE3), transfiziert. *E. coli* Stämme, die eine ausreichende lac Repressor Expression aufweisen, und somit induzierbar sind, sind geeignet. Das Chromosom der BL21 Bakterien enthält das Gen für die T7-RNA-Polymerase, welches vom lac-Promoter kontrolliert wird. Die Polymerase-Expression kann somit durch Isopropyl-β-D-thiogalactosid (IPTG) induziert werden. Das MIA-2 -Gen wird won einem T7-Promoter kontrolliert. Die Induktion mit IPTG führt folglich zur Expression von MIA-2.

Für die Anzucht des rekombinanten Bakterienstammes wird ein geeignetes Medium, wie z. B. LB-Medium, in einem geeignetem Volumen, i.d.R. 1,5 1, mit 100 µg/ml Ampicillin versetzt und mit einer Einzelkolonie beimpft.
**LB-Medium (11)**
10 g Trypton
5 g Hefeextrakt
10 g NaCl

Die Kultur wird bei 37 °C und 160-200 rpm geschüttelt. Bei einer OD₆₀₀ von 0.6-wird die Kultur mit 1 mM IPTG induziert und danach für weitere 4-5 Stunden bei 37 °C kultiviert bis Zelldichten der OD₆₀₀ bei 3-3,5 erzielt werden. Die Zellernte erfolgte durch Zentrifugation bei 10.000 g.

Das Bakterienpellet wird in 5ml Lysepuffer (0,1M NaPO4, 300mM NaCl, pH 7,5) aufgenommen und die Bakterien werden durch dreimaliges Einfrieren und 10min Ultraschallbehandlung aufgeschlossen. Die unlöslichen Bestandteile wurden durch Zentrifugation entfernt

### Reinigung von MIA-2

Das rekombinante Protein kann über chromatographische Reinigung des Fusionsproteins oder mittels chromatographischer Reinigungen, die die Eigenschaften von MIA-2 ausnutzen, gereinigt werden. Der Fusionsanteil kann mittels einer Protease abgespalten werden. Das Säulenmaterial mit dem gebundenen Protein wurde nun dreimal mit Lysepuffer und dreimal mit Waschpuffer (0,1M Na2PO4, 300mM NaCl, 20% Glyzerin, pH 6,1) gewaschen und anschließend in 3 ml PBS plus Protease aufgenommen. Die Suspension wird zur Spaltung zwischen MIA-2 und dem Fusionsanteil über Nacht bei 37 °C inkubiert. Nach dem Abzentrifugieren des Säulenmaterials wird der Überstand abgezogen, die MIA-2-Menge auf einem 20% SDS-PAGE Gel bestimmt und für die entsprechenden Versuche verwendet. Das Protein ist bei -20°C für mindestens einen Monat stabil. Die Reinheitsanalyse und Molekulargewichtsbestimmung wurde mit SDS-Polyacrylamidgelelektrophorese ermittelt.

### Beispiel 5: Rekombinante Expression von humanem MIA-2 in E. coli als Fusionsprotein

Die MIA-2 cDNA in einen *E. coli* Expressionsvektor kloniert, wie im Beispiel 4 beschrieben, nur dass zusätzlich ein Fusionsprotein wie z. B. DHFR, His-Tag oder GFP dabei ist. Um MIA-2 proteolytisch vom Fusionsprotein abtrennen zu können, wurde ein DNA Segment, das eine Erkennungsstelle z. B. für die IgA Protease (Ser Arg Pro Pro/Ser) zwischen Fusionsprotein und MIA-2 inseriert. Die Expression erfolgt analog wie im Beispiel 4 beschrieben. MIA-2 kann mittels des Fusionsproteins nach den üblichen Methoden gereinigt werden. Das rekombinante Protein kann über chromatographische Reinigung des Fusionsproteins oder nach chromatographischen Reinigungen, die die Eigenschaften von MIA-2 ausnutzen gereinigt werden. Das Säulenmaterial mit dem gebundenen Protein wird dreimal mit Lysepuffer und dreimal mit Waschpuffer (z. B. 0,1M Na₂PO₄, 300mM NaCl, 20% Glyzerin, pH 6,1) gewaschen. Nach dem Abzentrifugieren des Säulenmaterials wird der Überstand abgezogen, die MIA-2-Menge auf einem 20% SDS-PAGE Gel bestimmt und für die entsprechenden Versuche verwendet. Das Protein ist bei -20°C für mindestens einen Monat stabil. Die SDS-Polyacrylamidgelelektrophorese-Analyse ergab, dass das Protein in hoher Reinheit vorliegt.

### Beispiel 6: Nachweis von MIA-2 in verschiedenen Geweben und Zelllinien

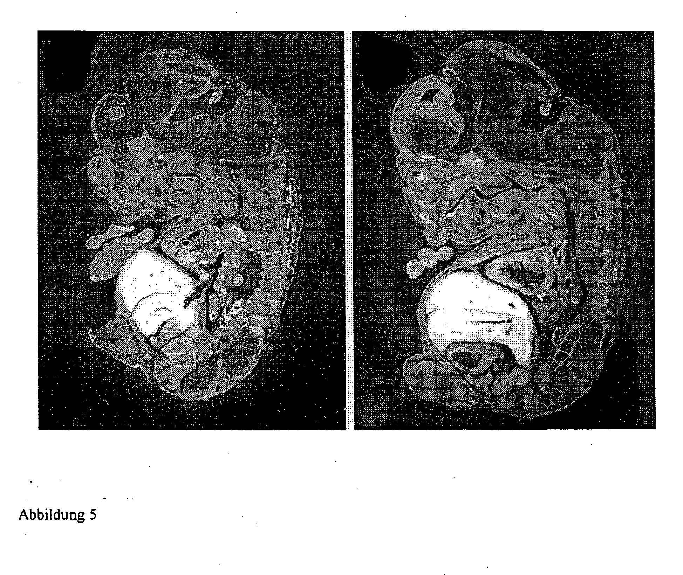

Zum einen kann die Expression von MIA-2 in bestimmten Zellen, genauer die MIA-2 mRNA, mit den üblichen Methoden der Nukleinsäurehybridisierung nachgewiesen werden, z.B. Northern Blot Analyse, in situ Hybridisierung, Dot oder Slot Blot Hybridisierung und davon abgeleitete Methoden (Sambrook et al., Molecular Cloning - A Laboratory Approach (1989), Cold Spring Harbor Laboratory Press; Nucleic Acid Hybridisation - A practical approach (1985), eds. B. D. Hames and S. J. Higgins, IRL Press; In situ Hybridisation - A practical approach (1993), ed. D. Wilkinson, IRL Press). Zum anderen kann mittels spezifischer Primer für MIA-2 die Expression durch RT-PCR (reverse transcriptase polymerase chain reaction) nachgewiesen werden (PCR Protocols - A guide to Methods and Applications (1990), eds. M. A. Innis, D. H. Gelfand, JJ. Sninsky, T. J. White, Academic Press INc; PCR - A practical approach (1991), eds. M. J. McPherson, P. Quire, G. R. Taylor (1991), IRL Press). Für die in situ Hybridisierung von MIA-2 auf Gewebedünnschnitten wurde eine P33-markierte Riboprobe aus den 390 N-terminalen Nukleotiden nach den üblichen Methoden hergestellt (siehe SEQ ID NO. 23). Nach der Hybridisierung und stringentem Waschen, wurden die Filme auf den Schnitten bis zu sechs Tagen exponiert. Die Abbildung 5 zeigt eine RNA in situ Hybridisierung von MIA-2 auf Gewebedünnschnitten von Mausembryonen (Embryonalstadium Tag 12,5 und 14,5). MIA-2 RNA wird spezifisch in der Leber exprimiert.

### Nachweis der MIA-2 RNA Expression mittlels RT-PCR

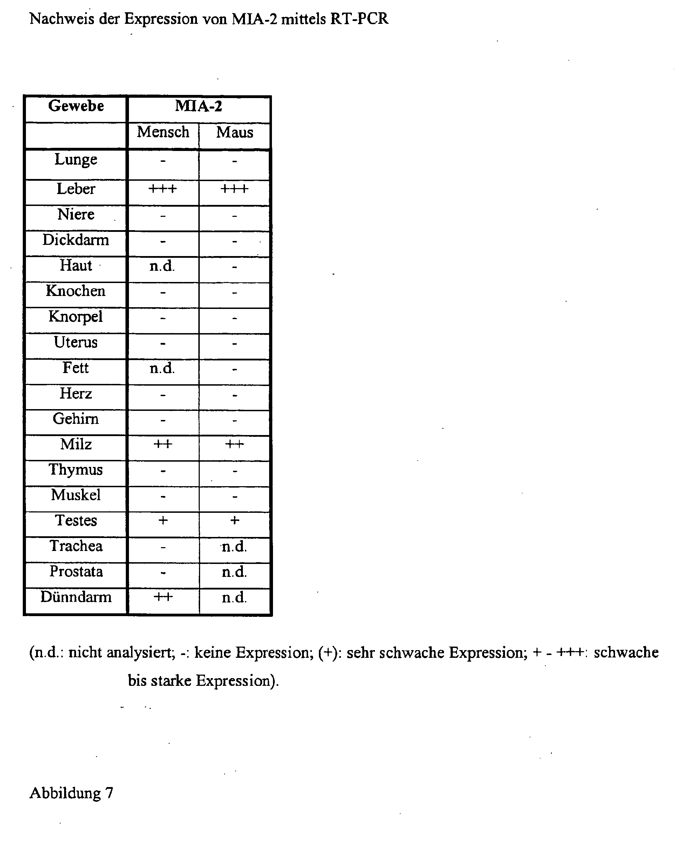

Abbildung 7 zeigt die MIA-2 RNA Expression in verschiedenen normalen Geweben von Maus und Mensch. Für die Untersuchung der gewebespezifichen Expression wurde Gesamt-RNA aus Gewebeproben von C57BL/6 Mäusen isoliert. Die Gesamt-RNA kann nach der Methode von Chomczynski und Sacchi, Anal. Biochem. 162 (1987) 156-159 oder mit handelsüblichen Kits, wie z. B. RNeasy kit (Qiagen, Hilden, Germany, Bestellnr. 75142) isoliert werden. Circa 0,4 cm³ Gewebe wird im Lysispuffer mittels dreier Gefrier- und Tauzyklen und Ultraschallbehandling homogenisiert. Die RNA wird aus dem Lysispuffer mit Hilfe von RNeasy Säulen abgetrennt. 1/10 der so erhaltenen RNA wird in der RT-PCR Analyse eingesetzt. Die humanen RNA Proben wurden von Clontech (Heidelberg, Germany) und Ambion (Austin, USA) bezogen. Die RNA wird mit Hilfe von dN6 Primem und einer reversen Transkriptase in cDNA umgeschrieben. Die Synthese des ersten cDNA Stranges erfolgte in einem Gesamtvolumen von 20 µl mit folgenden Komponenten: 2 µg Gesamt-RNA, 1 µg dN6 Primer (Pharmacia, Freiburg, Germany), 4 µl 5x erster Strang Puffer (Invitrogen Corporation, San Diego, USA), 2 µl 10mM DTT, 1 µl 10mM dNTPs und 1 µl Superscript Plus (Invitrogen Corporation, San Diego, USA). Die RT-PCR wurde semiquantitativ durchgeführt, wobei Primer für β-aktin (SEQ ID NO: 24 und 25) als Haushaltsgen zur Standardisierung verwendet wurden. Zur Standardisierung können auch andere Haushaltsgene wie z. B. Hypoxanthin-Phosphoribosyl-Transferase (HPRT, Transferrin Rezeptor, 18S RNA, Porphobilinogen Deaminase (PBGD), β2-microglobulin, 5-Aminolevulinat Synthase (ALAS) oder Glucose-6-Phosphat Dehydrogenase (GAPDH) verwendet werden. Alternativ zur klassischen oder semiquantitativen RT-PCR, kann auch eine quantitative PCR mit Hilfe des Lightcyclers (Roche Diagnostics, Mannheim, Germany) oder ABI PRISM® 7700 Sequence Detection System (Applied Biosystems, Foster City, CA, USA) durchgeführt werden.

Die klassische oder semiquantititative RT-PCR kann in jedem üblichen PCR Thermocycler, wie z. B. PTC-200 (Biozym, Hess. Oldendorf, Germany) oder 96-Well GeneAmp® PCR System 9700 (Applied Biosystems, Foster City, CA, USA) oder einem anderen Thermocycler durchgeführt werden. In einer typische RT-PCR Reaktion wurden 3 µl cDNA eingesetzt. Die PCR lief durch 32 Zyklen, wobei jeder Zyklus aus einer Denaturierungsphase (30 Sek. bei 94 °C), Annealingsphase (45 Sek. bei 58-62 °C) und Synthesephase (1 min bei 72 °C) bestand. Zum Schluß wurde die Reaktion noch 5 min bei 72 °C inkubiert. Die so erhaltenen PCR Produkte wurden auf einem 1,8 %igen Agarosegel elektrophoretisch aufgetrennt, mit Ethidiumbromid gefärbt und photographisch dokumentiert. Für MIA-2 spezifische RT-PCR wurden folgende Primer verwendet, für human SEQ ID NO. 3 (MIA-2 forward primer 5' ATGGCAAAATTTGGCGTTC) und SEQ ID NO. 26 (MIA-2 reverse primer 5' - CCTGCCCACAAATCTTCC) und für Maus SEQ ID NO. 3 (MIA-2 forward primer 5' -ATGGCAAAATTTGGCGTTC) und SEQ ID NO. 7 (MIA-2 reverse primer 5' - CCTGCCCACAAATCTTCT). MIA-2 wird im Menschen und in der Maus in den gleichen Geweben exprimiert, mit der stärksten Expression in der Leber.

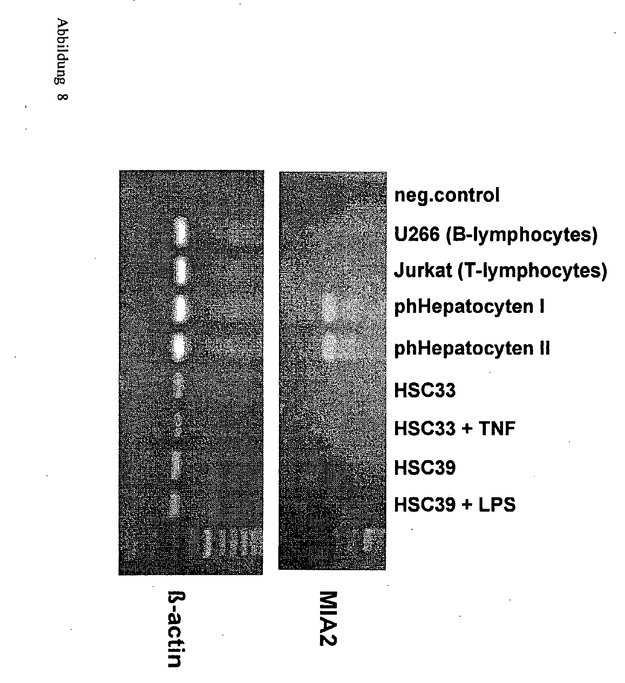

Mehrere Zellinien wurden bezüglich der MIA-2 Expression untersucht (Abbildung 8). MIA-2 wird ausschießlich in Hepatozyten stark exprimiert.

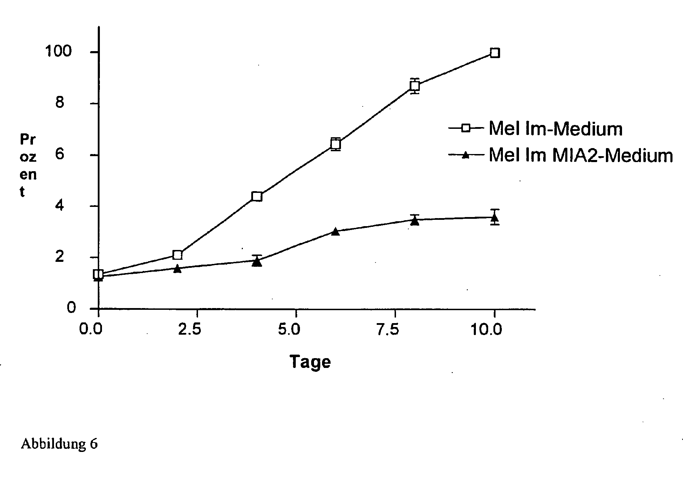

### Beispiel 7: Funktionelle Analysen der Wirkung von MIA-2 auf Ito-Zellen (Abbildung 6)

Um die Proliferation von getesteten Zellinien zu ermitteln, wurde deren Wachstum über 5 Tage durch Zellzählung verfolgt. Am Tag -1 werden die Zellen mit 2x10⁴ Zellen/Loch in einer 24-Loch-Platte ausgesät. An Tag 0 erfolgte die Behandlung mit MIA-2 (100 ng/ml - 5µg/ml) bzw. einer Kontrolle, an den folgenden Tagen werden die Zellen in je 2 Duplikaten gezählt. Die Bestimmung der Proliferation wird mit dem kommerziell verfügbaren XTT Test der Firma Roche (Kat. No. 1-465-015) durchgeführt. MIA-2 inhibiert die Proliferation der Itozellen.

### Beispiel 8: Nachweis der differentiellen MIA-2 Expression im Vergleich zwischen gesunden und veränderten Lebergewebe

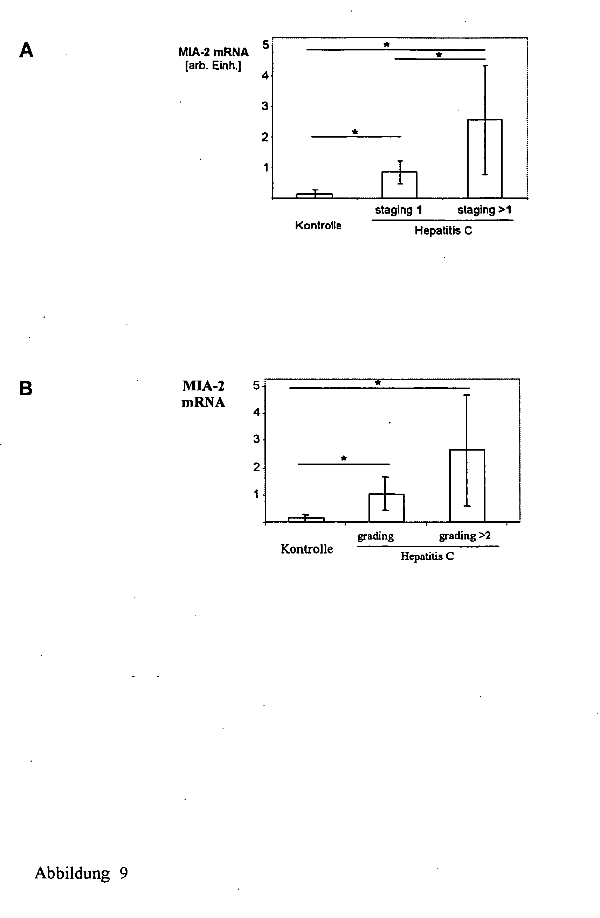

Mit Hilfe der im Beispiel 6 geschilderten Methodik kann die differentielle Genexpression von MIA-2 untersucht werden. Hierbei werden Gewebe, Gewebeflüssigkeiten und Blut (Serum) von Patienten mit unterschiedlichen Lebererkrankungen zur Analyse verwendet. RNA von Leberbiopsien von Patienten wurde isoliert und mittels RT-PCR analysiert. In Hepatitis Patienten mit milder Fibrose wird signifikant weniger MIA-2 exprimiert als in Hepatitis Patienten mit fortgeschrittener Fibrose (siehe Abbildung 9).

## Patentansprüche

1. Humanes MIA-2 Protein, das durch eine leberspezifische Expression und eine antiproliferative und antifibrotische Wirkung **gekennzeichnet** ist, und das von der Nukleinsäure von SEQ ID NO. 1 oder Varianten hiervon kodiert wird, wobei die Varianten eine oder mehrere Substitutionen, Insertionen und/oder Deletionen im Vergleich zur Nukleinsäure von SEQ ID NO. 1 und eine antiproliferative und antifibrotische Wirkung aufweisen, vorausgesetzt dass:
a) diese Varianten unter stringenten Bedingungen an eine Nukleinsäure gemäß der Sequenz von SEQ ID NO. 1 hybridisieren und weiterhin vorausgesetzt, dass diese Varianten ein Protein mit einer MIA-2-Aktivität kodieren, oder
b) diese Varianten Nukleinsäureveränderungen aufweisen, die auf die Degenerierung des genetischen Codes zurückzuführen sind, die für dieselbe Aminosäure oder eine Aminosäure mit derselben Aktivität wie die Nukleinsäure von SEQ ID NO. 1 kodieren, und
c) das MIA-2-Protein jeweils nicht ausschließlich durch die Basen 1-354 von SEQ ID NO. 1 oder Bruchstücken hiervon kodiert wird.

2. Murines MIA-2 Protein, das durch eine leberspezifische Expression und eine antiproliferative und antifibrotische Wirkung **gekennzeichnet** ist, und das von der Nukleinsäure von SEQ ID NO. 27 oder Varianten hiervon kodiert wird, wobei die Varianten eine oder mehrere Substitutionen, Insertionen und/oder Deletionen im Vergleich zur Nukleinsäure von SEQ ID NO. 27 und eine antiproliferative und antifibrotische Wirkung aufweisen, vorausgesetzt dass:
a) diese Varianten unter stringenten Bedingungen an eine Nukleinsäure gemäß der Sequenz von SEQ ID NO. 27 hybridisieren und weiterhin vorausgesetzt, dass diese Varianten ein Protein mit einer MIA-2-Aktivität kodieren, oder
b) diese Varianten Nukleinsäureveränderungen aufweisen, die auf die Degenerierung des genetischen Codes zurückzuführen sind, die für dieselbe Aminosäure oder eine Aminosäure mit derselben Aktivität wie die Nukleinsäure von SEQ ID NO. 27 kodieren, und
c) das MIA-2-Protein jeweils nicht ausschließlich durch die Basen 1-357 von SEQ ID NO. 27 oder Bruchstücken hiervon kodiert wird.

3. Isolierte Nukleinsäure, die die Nukleinsäure von SEQ ID NO. 1 oder Varianten hiervon umfasst, wobei die Varianten eine oder mehrere Substitutionen, Insertionen und/oder Deletionen im Vergleich zur Nukleinsäure von SEQ ID NO. 1 aufweisen, und für ein Protein kodieren, das durch eine leberspezifische Expression und eine antiproliferative und antifibrotische Wirkung **gekennzeichnet** ist, vorausgesetzt dass:
a) diese Varianten unter stringenten Bedingungen an eine Nukleinsäure gemäß der Sequenz von SEQ ID NO. 1 hybridisieren und weiterhin vorausgesetzt, dass diese Varianten ein Protein mit einer MIA-2-Aktivität kodieren, oder
b) diese Varianten Nukleinsäureveränderungen aufweisen, die auf die Degenerierung des genetischen Codes zurückzuführen sind, die für dieselbe Aminosäure oder eine Aminosäure mit derselben Aktivität wie die Nukleinsäure von SEQ ID NO. 1 kodieren, und
c) die Nukleinsäure nicht ausschließlich aus den Basen 1-354 von SEQ ID NO. 1 oder Bruchstücken hiervon besteht.

4. Isolierte Nukleinsäure, die die Nukleinsäure von SEQ ID NO. 27 oder Varianten hiervon umfasst, wobei die Varianten eine oder mehrere Substitutionen, Insertionen und/oder Deletionen im Vergleich zur Nukleinsäure von SEQ ID NO. 27 aufweisen, und für ein Protein kodieren, das durch eine leberspezifische Expression und eine antiproliferative und antifibrotische Wirkung **gekennzeichnet** ist, vorausgesetzt dass:
a) diese Varianten unter stringenten Bedingungen an eine Nukleinsäure gemäß der Sequenz von SEQ ID NO. 27 hybridisieren und weiterhin vorausgesetzt, dass diese Varianten ein Protein mit einer MIA-2-Aktivität kodieren, oder
b) diese Varianten Nukleinsäureveränderungen aufweisen, die auf die Degenerierung des genetischen Codes zurückzuführen sind, die für dieselbe Aminosäure oder eine Aminosäure mit derselben Aktivität wie die Nukleinsäure von SEQ ID NO. 27 kodieren, und
c) die Nukleinsäure nicht ausschließlich aus den Basen 1-357 von SEQ ID NO. 27 oder Bruchstücken hiervon besteht.

5. Isolierte Nukleinsäure nach Anspruch 3 oder 4, die weiterhin mit einer oder mehreren regulatorischen Sequenzen in funktioneller Weise verbunden ist, wobei als regulatorische Sequenz vorzugsweise der humane MIA-2-Promotor nach SEQ ID NO. 2 eingesetzt wird.

6. MIA-2 Promotor, der die Nukleinsäuresequenz von SEQ ID NO. 2 aufweist.

7. Nukleinsäure, die ein Transkriptionsprodukt einer der Nukleinsäuren der Ansprüche 3-5 ist oder unter stringenten Bedingungen selektiv an ein solches Transkriptionsprodukt hybridisiert und eine Antisense DNA oder RNA ist.

8. Humanes MIA-2 Protein, das die Aminosäure von SEQ ID NO. 5 oder eine Variante dieser Aminosäure umfasst, wobei die Varianten eine oder mehrere Substitutionen, Insertionen und/oder Deletionen im Vergleich zur Aminosäure von SEQ ID NO. 5 aufweisen, und wobei die biologische Aktivität des Proteins der Aktivität des MIA-2 Proteins, das die unmodifizierte Sequenz von SEQ ID NO. 5 umfasst, im wesentlichen gleich ist und durch eine leberspezifische Expression und eine antiproliferative und antifibrotische Wirkung **gekennzeichnet** ist, wobei die Varianten nicht ausschließlich aus den Aminosäureresten 1-118 von SEQ ID NO. 5 bestehen.

9. Murines MIA-2 Protein, das die Aminosäure von SEQ ID NO. 28 oder eine Variante dieser Aminosäure umfasst, wobei die Varianten eine oder mehrere Substitutionen, Insertionen und/oder Deletionen im Vergleich zur Aminosäure von SEQ ID NO. 28 aufweisen, und wobei die biologische Aktivität des Proteins der Aktivität des MIA-2 Proteins, das die unmodifizierte Sequenz von SEQ ID NO. 28 umfasst, im wesentlichen gleich ist, und durch eine leberspezifische Expression und eine antiproliferative und antifibrotische Wirkung **gekennzeichnet** ist, wobei die Varianten nicht ausschließlich aus den Aminosäureresten 1-119 von SEQ ID NO. 28 bestehen.

10. Vektor, der eine Nukleinsäure nach einem oder mehreren der Ansprüche 3-7 umfasst und vorzugsweise ein Plasmid ist.

11. Wirtszelle, die mit einem Vektor nach Anspruch 10 transformiert worden ist und vorzugsweise eine eukaryontische Zelle, besonders bevorzugt eine Säugetierzelle, vorzugsweise eine adulte oder embryonale Stammzelle, Pflanzenzelle, Hefezelle oder Insektenzelle, noch mehr bevorzugt eine CHO, COS, HeLa, 293T, HEH oder BHK-Zelle, oder eine prokaryontische Zelle, vorzugsweise E. coli oder Bacillus subtilis, ist,
wobei menschliche, embryonale Stammzellen ausgenommen sind.

12. Antikörper oder Aptamer, das für ein Epitop des MIA-2 Proteins nach Anspruch 2 oder 9 spezifisch ist und der Antikörper vorzugsweise aus der Gruppe ausgewählt ist, die aus einem polyklonalen Antikörper, einem monoklonalen Antikörper, einem humanisierten Antikörper, einem chimären Antikörper und einem synthetischen Antikörper besteht und wahlweise an ein toxisches und/oder nachweisbares Mittel gebunden ist.

13. Pharmazeutische Zusammensetzung, die eine therapeutisch wirksame Dosis einer Nukleinsäure nach einem oder mehreren der Ansprüche 3-7 oder eines Vektors nach Anspruch 10 in Kombination mit einem pharmazeutisch verträglichen Träger, oder eine therapeutisch wirksame Dosis eines Proteins nach Anspruch 1 oder 8 in Kombination mit einem pharmazeutisch verträglichen Träger und wahlweise weitere Wirkstoffe wie z.B. Interferone, Inhibitoren des ACE-Systems oder Liganden des proliferation-activated receptor-g (PPAR-g), umfasst.

14. Pharmazeutische oder diagnostische Zusammensetzung, die einen Antikörper oder ein Aptamer nach Anspruch 12 umfasst.

15. Transgenes, nicht-menschliches Säugetier, vorzugsweise transgene Maus, bei dem eine Nukleinsäure nach Anspruch 4, vorzugsweise konditionell, inaktiviert wurde, oder in dessen Genom eine Nukleinsäure nach Anspruch 6 eingefügt wurde.

16. Ex vivo-Verfahren zur Diagnose einer Leberschädigung oder zur Bestimmung der hepatischen Syntheseleistung, das folgende Schritte umfasst:
a) Bereitstellung einer Lebergewebe- oder Serumprobe oder einer Biopsie von einem Patienten,
b) Qualitative und/oder quantitative Bestimmung der Transkriptionsprodukte nach Anspruch 7, oder der Transkriptionsprodukte einer Nukleinsäure, die die Nukleinsäure von SEQ ID NO. 1 oder 27 oder Varianten hiervon umfasst, wobei die Varianten eine oder mehrere Substitutionen, Insertionen und/oder Deletionen im Vergleich zur Nukleinsäure von SEQ ID NO. 1 oder 27 aufweisen, vorausgesetzt dass:
- diese Varianten unter stringenten Bedingungen an eine Nukleinsäure hybridisieren, die die Sequenz von SEQ ID NO. 1 oder 27 umfasst und weiterhin vorausgesetzt, dass diese Varianten ein Protein mit einer MIA-2-Aktivität kodieren, oder
- diese Varianten Nukleinsäureveränderungen aufweisen, die auf die Degenerierung des genetischen Codes zurückzuführen sind, die für dieselbe oder eine funktionell äquivalente Aminosäure wie die Nukleinsäure von SEQ ID NO. 1 oder 27 kodieren,
in der Probe,
wobei eine Überexpression der Transkriptionsprodukte auf eine Leberschädigung und/oder eine gesteigerte hepatische Syntheseleistung hinweist.

17. Verfahren nach Anspruch 16, wobei die Bestimmung in Schritt b) durch Northern Blot, in situ Hybridisierung oder RT-PCR, wobei eine RT-PCR vorzugsweise mit den Primern nach SEQ ID NO. 3, 4, 7, 9, 26 oder 29-36 durchgeführt wird, oder einer Kombination hiervon erfolgt, oder
wobei die Bestimmung in Schritt b) unter Verwendung einer Zusammensetzung nach Anspruch 14 erfolgt.

18. Ex vivo-Verfahren zur Diagnose einer Leberschädigung oder zur Bestimmung der hepatischen Syntheseleistung , das folgende Schritte umfasst:
a) Bereitstellung einer Lebergewebe- oder Serumprobe oder Biopsie von einem Patienten,
b) in Berührung bringen der Probe mit einem Reagenz, das zum Nachweis des MIA-2-Proteins nach Anspruch 8 oder 9 in der Lage ist, vorzugsweise mit einem Antikörper nach Anspruch 12 oder einem Antikörper, der für ein Epitop des MIA-2 Proteins nach Anspruch 1 oder 8 spezifisch ist,
c) Qualitative und/oder quantitative Bestimmung des MIA-2-Proteins in der Probe,
wobei das Vorliegen und die Menge des MIA-2-Proteins auf eine Leberschädigung und/oder eine gesteigerte hepatische Syntheseleistung hinweist.

19. Verfahren nach einem oder mehreren der Ansprüche 16-18, bei dem die Leberschädigung eine Leberzirrhose, Leberfibrose oder ein Lebertumor/-Metastase ist.

20. Verwendung der pharmazeutischen Zusammensetzung nach Anspruch 13 zur Herstellung eines Arzneimittels für die antifibrotische Therapie oder für die Behandlung der Leberzirrhose oder Leberfibrose.

21. Verwendung der pharmazeutischen Zusammensetzung nach Anspruch 13 und/oder 14 zur Herstellung eines Arzneimittels für die Behandlung von Lebertumoren/- Metastasen.

22. Verwendung eines Antikörpers nach Anspruch 12 oder eines Antikörpers, der gegen ein Epitop des MIA-2 Proteins nach Anspruch 1 oder 8 gerichtet ist, zur ex-vivo Diagnose einer Leberschädigung oder zur Bestimmung der hepatischen Syntheseleistung.

23. Verwendung nach Anspruch 22 bei der die Leberschädigung eine Leberzirrhose, Leberfibrose oder ein Lebertumor/-Metastase ist.

## Claims

1. A human MIA-2 protein **characterised by** a liver-specific expression and an anti-proliferative and anti-fibrotic effect and encoded by the nucleic acid of SEQ ID NO: 1 or variants thereof, wherein the variants have one or more substitutions, insertions and/or deletions as compared to the nucleic acid of SEQ ID NO: 1 and an anti-proliferative and anti-fibrotic effect, provided that
a) these variants hybridise with a nucleic acid according to the sequence of SEQ ID NO: 1 under stringent conditions, and further provided that these variants encode a protein having MIA-2 activity, or
b) these variants have nucleic acid changes due to the degeneration of the genetic code and encoding the same amino acid or an amino acid having the same activity as the nucleic acid of SEQ ID NO: 1, and
c) the MIA-2 protein each is not exclusively encoded by the bases 1-354 of SEQ ID NO: 1 or fragments thereof.

2. A murine MIA-2 protein **characterised by** a liver-specific expression and an anti-proliferative and anti-fibrotic effect and encoded by the nucleic acid of SEQ ID NO: 27 or variants thereof, wherein the variants have one or more substitutions, insertions and/or deletions as compared to the nucleic acid of SEQ ID NO: 27 and an anti-proliferative and anti-fibrotic effect, provided that
a) these variants hybridise with a nucleic acid according to the sequence of SEQ ID NO: 27 under stringent conditions, and further provided that these variants encode a protein having MIA-2 activity, or
b) these variants have nucleic acid changes due to the degeneration of the genetic code and encoding the same amino acid or an amino acid having the same activity as the nucleic acid of SEQ ID NO: 27, and
c) the MIA-2 protein each is not exclusively encoded by the bases 1-357 of SEQ ID NO: 27 or fragments thereof.

3. An isolated nucleic acid comprising the nucleic acid of SEQ ID NO:1 or variants thereof, wherein the variants have one or more substitutions, insertions and/or deletions as compared to the nucleic acid of SEQ ID NO:1 and encode a protein **characterised by** a liver-specific expression and an anti-proliferative and anti-fibrotic effect, provided that:
a) these variants hybridise with a nucleic acid according to the sequence of SEQ ID NO: 1 under stringent conditions, and further provided that these variants encode a protein having MIA-2 activity, or
b) these variants have nucleic acid changes due to the degeneration of the genetic code and encoding the same amino acid or an amino acid having the same activity as the nucleic acid of SEQ ID NO: 1, and
c) the nucleic acid does not exclusively consist of the bases 1-354 of SEQ ID NO: 1 or fragments thereof.

4. An isolated nucleic acid comprising the nucleic acid of SEQ ID NO: 27 or variants thereof, wherein the variants have one or more substitutions, insertions and/or deletions as compared to the nucleic acid of SEQ ID NO: 27 and encode a protein **characterised by** a liver-specific expression and an anti-proliferative and anti-fibrotic effect, provided that:
a) these variants hybridise with a nucleic acid according to the sequence of SEQ ID NO: 27 under stringent conditions, and further provided that these variants encode a protein having MIA-2 activity, or
b) these variants have nucleic acid changes due to the degeneration of the genetic code and encoding the same amino acid or an amino acid having the same activity as the nucleic acid of SEQ ID NO: 27, and
c) the nucleic acid does not exclusively consist of the bases 1-357 of SEQ ID NO: 27 or fragments thereof.

5. The isolated nucleic acid according to claim 3 or 4 further functionally linked with one or more regulatory sequences, wherein the human MIA-2 promoter according to SEQ ID NO: 2 is preferably used as the regulatory sequence.

6. A MIA-2 promoter having the nucleic acid sequence of SEQ ID NO: 2.

7. A nucleic acid which is a transcriptional product of one of the nucleic acids according to claims 3-5 or which selectively hybridises with such a transcription product under stringent conditions and is an *antisense* DNA or RNA.

8. A human MIA-2 protein which comprises the amino acid of SEQ ID NO: 5 or a variant of this amino acid, wherein the variants have one or more substitutions, insertions and/or deletions as compared to the amino acid of SEQ ID NO: 5 and wherein the biological activity of the protein is substantially the same as the activity of the MIA-2 protein comprising the unmodified sequence of SEQ ID NO: 5, and which is **characterised by** a liver-specific expression and an anti-proliferative and anti-fibrotic effect, wherein the variants do not exclusively consist of the amino acid residues 1-118 of SEQ ID NO: 5.

9. A murine MIA-2 protein which comprises the amino acid of SEQ ID NO: 28 or a variant of this amino acid, wherein the variants have one or more substitutions, insertions and/or deletions as compared to the amino acid of SEQ ID NO: 28 and wherein the biological activity of the protein is substantially the same as the activity of the MIA-2 protein comprising the unmodified sequence of SEQ ID NO: 28, and which is **characterised by** a liver-specific expression and an anti-proliferative and anti-fibrotic effect, wherein the variants do not exclusively consist of the amino acid residues 1-119 of SEQ ID NO: 28.

10. A vector which comprises a nucleic acid according to one or more of the claims 3-7 and which is preferably a plasmid.

11. A host cell which has been transformed with a vector according to claim 10 and which is preferably an eukaryotic cell, more preferably a mammal cell, preferably an adult or embryonic stem cell, plant cell, yeast cell or insect cell, even more prefereably a CHO, COS, HeLa, 293T, HEH or BHK cell, or a prokaryotic cell, preferably E. coli or Bacillus subtilis, wherein human embryonic stem cells are excluded.

12. An antibody or an aptamer which is specific for an epitope of the MIA-2 protein according to claim 2 or 9, wherein the antibody is preferably selected from the group consisting of a polyclonal antibody, a monoclonal antibody, a humanised antibody, a chimeric antibody and a synthetic antibody, and is optionally bound to a toxic and/or a detectable substance.

13. A pharmaceutical composition comprising a therapeutically effective dose of a nucleic acid according to one or more of the claims 3-7 or of a vector according to claim 10 in combination with a pharmaceutically acceptable carrier, or a therapeutically effective dose of a protein according to claim 1 or 8 in combination with a pharmaceutically acceptable carrier and optionally other active substances as e.g. interferones, inhibitors of the ACE system or ligands of the proliferation-activated receptor-g (PPAR-g).

14. A pharmaceutical or diagnostic composition comprising an antibody or an aptamer according to claim 12.

15. A transgenic, non-human mammal, preferably a transgenic mouse, in which one nucleic acid according to claim 4 was, preferably conditionally, inactivated or which has one nucleic acid according to claim 6 inserted in its genome.

16. An *ex vivo* method for the diagnosis of a liver damage or for the determination of the hepatic synthesis performance, comprising the following steps:
a) providing a liver tissue or serum sample or a biopsy of a patient,
b) qualitatively and/or quantitatively determining the transcription products according to claim 7, or
the transcription products of a nucleic acid comprising the nucleic acid of SEQ ID NO: 1 or 27 or variants thereof, wherein the variants have one or more substitutions, insertions and/or deletions as compared to the nucleic acid of SEQ ID NO: 1 or 27, provided that:
- these variants hybridise with a nucleic acid comprising the sequence of SEQ ID NO: 1 or 27 under stringent conditions, and further provided that these variants encode a protein having MIA-2 activity, or
- these variants have nucleic acid changes due to the degeneration of the genetic code and encoding the same amino acid or an amino acid functionally equivalent to the nucleic acid of SEQ ID NO: 1 or 27,
in the sample,
wherein an overexpression of the transcription products indicates a liver damage and/or an increased hepatic synthesis performance.

17. The method according to claim 16, wherein the determination in step b) is carried out by the use of Northern Blot, in situ hybridisation or RT PCR, wherein the RT PCR is preferably carried out by using the primers of SEQ ID NO: 3, 4, 7, 9, 26 or 29-36 or a combination thereof, or
wherein the determination in step b) is carried out with the use of a composition according to claim 14.

18. An *ex vivo* method for the diagnosis of a liver damage or for the determination of the hepatic synthesis performance, comprising the following steps:
a) providing a liver tissue or serum sample or a biopsy of a patient,
b) contacting the sample with a reagent capable of detecting the MIA-2 protein according to claim 8 or 9, preferably by using an antibody according to claim 12 or an antibody which is specific for an epitope of the MIA-2 protein according to claim 1 or 8,
c) qualitatively and/or quantitatively determining the MIA-2 protein in the sample,
wherein the presence and the amount of the MIA-2 protein indicate a liver damage and/or an increased hepatic synthesis performance.

19. The method according to one or more of the claims 16-18, wherein the liver damage is cirrhosis of the liver, hepatic fibrosis or a liver tumor/metastasis.

20. Use of the pharmaceutical composition according to claim 13 for the manufacture of a medicament for the anti-fibrotic therapy or for the treatment of cirrhosis of the liver or hepatic fibrosis.

21. Use of the pharmaceutical composition according to claim 13 and/or 14 for the manufacture of a medicament for the treatment of liver tumors/metastases.

22. Use of an antibody according to claim 12 or an antibody directed against an epitope of the MIA-2 protein according to claim 1 or 8 for the *ex vivo* diagnosis of a liver damage or for the determination of the hepatic synthesis performance.

23. Use according to claim 22, wherein the liver damage is cirrhosis of the liver, hepatic fibrosis or a liver tumor/metastasis.

## Revendications

1. Protéine MIA-2 humaine qui est **caractérisée par** une expression spécifique au foie et par une action antiproliférative et antifibrotique et qui est codée par l'acide nucléique de séquence SEQ ID n° 1 ou par des variantes de celui-ci, les variantes comportant une ou plusieurs substitutions, insertions et/ou délétions en comparaison de l'acide nucléique de séquence SEQ ID n° 1 et présentant une action antiproliférative et antifibrotique, à condition que :
a) ces variantes soient hybridées dans des conditions strictes à un acide nucléique selon la séquence SEQ ID n° 1 et ces variantes codent une protéine ayant une activité MIA-2, ou
b) ces variantes comportent des modifications d'acide nucléique qui sont à attribuer à la dégénération du code génétique et qui codent le même aminoacide ou un aminoacide ayant la même activité que l'acide nucléique de séquence SEQ ID n° 1, et
c) la protéine MIA-2 ne soit pas codée à chaque fois exclusivement par les bases 1 à 354 de la séquence SEQ ID n° 1 ou par des fragments de celles-ci.

2. Protéine MIA-2 murine, qui est **caractérisée par** une expression spécifique au foie et par une action antiproliférative et antifibrotique et qui est codée par l'acide nucléique de séquence SEQ ID n° 27 ou par des variantes de celui-ci, les variantes comportant une ou plusieurs substitutions, insertions et/ou délétions en comparaison de l'acide nucléique de séquence SEQ ID n° 27 et présentant une action antiproliférative et antifibrotique, à condition que :
a) ces variantes soient hybridées dans des conditions strictes à un acide nucléique selon la séquence SEQ ID n° 27 et ces variantes codent une protéine ayant une activité MIA-2, ou
b) ces variantes comportent des modifications d'acide nucléique qui sont à attribuer à la dégénération du code génétique et qui codent le même aminoacide ou un aminoacide ayant la même activité que l'acide nucléique de séquence SEQ ID n° 27, et
c) la protéine MIA-2 ne soit pas codée à chaque fois exclusivement par les bases 1 à 357 de la séquence SEQ ID n° 27 ou par des fragments de celles-ci.

3. Acide nucléique isolé, qui comprend l'acide nucléique de séquence SEQ ID n° 1 ou des variantes de celui-ci, les variantes comportant une ou plusieurs substitutions, insertions et/ou délétions en comparaison de l'acide nucléique de séquence SEQ ID n° 1 et codant une protéine qui est **caractérisée par** une expression spécifique au foie et par une action antiproliférative et antifibrotique, à condition que :
a) ces variantes soient hybridées dans des conditions strictes à un acide nucléique selon la séquence SEQ ID n° 1 et ces variantes codent une protéine ayant une activité MIA-2, ou
b) ces variantes comportent des modifications d'acide nucléique qui sont à attribuer à la dégénération du code génétique et qui codent le même aminoacide ou un aminoacide ayant la même activité que l'acide nucléique de séquence SEQ ID n° 1, et
c) l'acide nucléique ne soit pas constitué exclusivement des bases 1 à 354 de la séquence SEQ ID n° 1 ou de fragments de celles-ci.

4. Acide nucléique isolé, qui comprend l'acide nucléique de séquence SEQ ID n° 27 ou des variantes de celui-ci, les variantes comportant une ou plusieurs substitutions, insertions et/ou délétions en comparaison de l'acide nucléique de séquence SEQ ID n° 27 et codant une protéine qui est **caractérisée par** une expression spécifique au foie et par une action antiproliférative et antifibrotique, à condition que :
a) ces variantes soient hybridées dans des conditions strictes à un acide nucléique selon la séquence SEQ ID n° 27 et ces variantes codent une protéine ayant une activité MIA-2, ou
b) ces variantes comportent des modifications d'acide nucléique qui sont à attribuer à la dégénération du code génétique et qui codent le même aminoacide ou un aminoacide ayant la même activité que l'acide nucléique de séquence SEQ ID n° 27, et
c) l'acide nucléique ne soit pas constitué exclusivement des bases 1 à 357 de la séquence SEQ ID n° 27 ou de fragments de celles-ci.

5. Acide nucléique isolé selon la revendication 3 ou 4, qui est aussi relié fonctionnellement à une ou plusieurs séquences régulatrices, le promoteur MIA-2 humain selon la séquence SEQ ID n° 2 étant utilisé de préférence comme séquence régulatrice.

6. Promoteur MIA-2 qui comporte la séquence d'acide nucléique SEQ ID n° 2.

7. Acide nucléique qui est un produit de transcription de l'un des acides nucléiques des revendications 3 à 5 ou qui est hybridé dans des conditions strictes et de manière sélective à un produit de transcription de ce type et qui est un ADN ou un ARN antisens.

8. Protéine MIA-2 humaine qui comprend l'aminoacide de séquence SEQ ID n° 5 ou une variante de cet aminoacide, les variantes comportant une ou plusieurs substitutions, insertions et/ou délétions en comparaison de l'aminoacide de séquence SEQ ID n° 5 et l'activité biologique de la protéine étant globalement identique à l'activité de la protéine MIA-2 qui comprend la séquence non modifiée SEQ ID n° 5 et étant **caractérisée par** une expression spécifique au foie et par une action antiproliférative et antifibrotique, les variantes n'étant pas constituées exclusivement des résidus aminoacides 1 à 118 de la séquence SEQ ID n° 5.

9. Protéine MIA-2 murine qui comprend l'aminoacide de séquence SEQ ID n° 28 ou une variante de cet aminoacide, les variantes comportant une ou plusieurs substitutions, insertions et/ou délétions en comparaison de l'aminoacide de séquence SEQ ID n° 28 et l'activité biologique de la protéine étant globalement identique à l'activité de la protéine MIA-2 qui comprend la séquence non modifiée SEQ ID n° 28 et étant **caractérisée par** une expression spécifique au foie et par une action antiproliférative et antifibrotique, les variantes n'étant pas constituées exclusivement des résidus aminoacides 1 à 119 de la séquence SEQ ID n° 28.

10. Vecteur qui comprend un acide nucléique selon une ou plusieurs des revendications 3 à 7 et qui est de préférence un plasmide.

11. Cellule hôte qui a été transformée avec un vecteur selon la revendication 10 et qui est de préférence une cellule eucaryote, particulièrement de préférence une cellule de mammifère, de préférence une cellule souche adulte ou embryonnaire, une cellule de plante, une cellule de levure ou une cellule d'insecte, et encore plus de préférence une cellule CHO, COS, HeLa, 293T, HEH ou BHK, ou une cellule procaryote, de préférence E. coli ou Bacillus subtilis, les cellules souches humaines, embryonnaires étant exclues.

12. Anticorps ou aptamère qui est spécifique à un épitope de la protéine MIA-2 selon la revendication 2 ou 9, l'anticorps étant choisi de préférence dans le groupe qui est constitué d'un anticorps polyclonal, d'un anticorps monoclonal, d'un anticorps humanisé, d'un anticorps chimère et d'un anticorps synthétique et étant lié au choix à un agent toxique et/ou à un agent décelable.

13. Composé pharmaceutique qui comprend une dose active d'un point de vue thérapeutique d'un acide nucléique selon une ou plusieurs des revendications 3 à 7 ou d'un vecteur selon la revendication 10 en combinaison avec un support acceptable d'un point de vue pharmaceutique ou une dose active d'un point de vue thérapeutique d'une protéine selon la revendication 1 ou 8 en combinaison avec un support acceptable d'un point de vue pharmaceutique et au choix d'autres principes actifs tels que par exemple interférons, inhibiteurs du système ACE ou ligandes du récepteur g activé par proliferation (PPAR-g).

14. Composé pharmaceutique ou de diagnostic qui comprend un anticorps ou un aptamère selon la revendication 12.

15. Mammifère transgénique non humain, de préférence souris transgénique, dans lequel a été inactivé un acide nucléique selon la revendication 4 de préférence de manière conditionnelle ou dans le génome duquel a été inséré un acide nucléique selon la revendication 6.

16. Procédé ex vivo pour le diagnostic d'une lésion du foie ou pour la détermination de la capacité de synthèse hépatique qui comprend les étapes suivantes :
a) préparation d'un échantillon de sérum ou de tissu hépatique ou d'une biopsie d'un patient,
b) détermination qualitative et/ou quantitative des produits de transcription selon la revendication 7 ou des produits de transcription d'un acide nucléique qui comprend l'acide nucléique de séquence SEQ ID n° 1 ou 27 ou des variantes de celui-ci, les variantes comportant une ou plusieurs substitutions, insertions et/ou délétions en comparaison de l'acide nucléique de séquence SEQ ID n° 1 ou 27, à condition que :
- ces variantes soient hybridées dans des conditions strictes à un acide nucléique qui comprend la séquence SEQ ID n° 1 ou 27 et ces variantes codent une protéine ayant une activité MIA-2, ou
- ces variantes comportent des modifications d'acide nucléique qui sont à attribuer à la dégénération du code génétique et qui codent le même aminoacide ou un aminoacide fonctionnellement équivalent à l'acide nucléique de séquence SEQ ID n° 1 ou 27,
dans l'échantillon,
une surexpression des produits de transcription trahissant une lésion du foie et/ou une augmentation de la capacité de synthèse hépatique.

17. Procédé selon la revendication 16, selon lequel la détermination à l'étape b) s'effectue par un buvardage de Northern, hybridation in situ ou RT-PCR, une RT-PCR étant effectuée de préférence avec les amorces selon SEQ ID n° 3, 4, 7, 9, 26 ou 29 à 36, ou par une combinaison de ces techniques, ou
selon lequel la détermination à l'étape b) s'effectue en utilisant un composé selon la revendication 14.

18. Procédé ex vivo pour le diagnostic d'une lésion du foie ou pour la détermination de la capacité de synthèse hépatique qui comprend les étapes suivantes :
a) préparation d'un échantillon de sérum ou de tissu hépatique ou d'une biopsie d'un patient,
b) mise en contact de l'échantillon avec un réactif qui est capable de déceler la protéine MIA-2 selon la revendication 8 ou 9, de préférence avec un anticorps selon la revendication 12 ou avec un anticorps qui est spécifique à un épitope de la protéine MIA-2 selon la revendication 1 ou 8,
c) détermination qualitative et/ou quantitative de la protéine MIA-2 dans l'échantillon,
la présence et la quantité de la protéine MIA-2 trahissant une lésion du foie et/ou une augmentation de la capacité de synthèse hépatique.

19. Procédé selon une ou plusieurs des revendications 16 à 18, selon lequel la lésion du foie est une cirrhose hépatique, une fibrose hépatique ou une métastase / tumeur hépatique.

20. Utilisation du composé pharmaceutique selon la revendication 13 en vue de la fabrication d'un médicament pour la thérapie antifibrotique ou pour le traitement de la cirrhose hépatique ou de la fibrose hépatique.

21. Utilisation du composé pharmaceutique selon la revendication 13 et/ou 14 en vue de la fabrication d'un médicament pour le traitement de métastases / tumeurs hépatiques.

22. Utilisation d'un anticorps selon la revendication 12 ou d'un anticorps qui est dirigé contre un épitope de la protéine MIA-2 selon la revendication 1 ou 8 pour le diagnostic ex vivo d'une lésion du foie ou pour la détermination de la capacité de synthèse hépatique.

23. Utilisation selon la revendication 22 selon laquelle la lésion du foie est une cirrhose hépatique, une fibrose hépatique ou une métastase / tumeur hépatique.
